(19) Europäisches Patentamt
European Patent Office
Office européen des brevets

(11) Veröffentlichungsnummer: **0 049 760**
**A2**

(12) **EUROPÄISCHE PATENTANMELDUNG**

(21) Anmeldenummer: 81107019.2

(22) Anmeldetag: 07.09.81

(51) Int. Cl.³: C 07 D 271/02
C 07 D 285/04, C 07 D 231/12
C 07 D 275/02, C 07 D 277/12
C 07 D 261/08, C 07 D 307/52
A 01 N 25/32

(30) Priorität: 19.09.80 DE 3035356

(43) Veröffentlichungstag der Anmeldung: 21.04.82 Patentblatt 82/16

(84) Benannte Vertragsstaaten: BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Ditgens, Klaus, Dr.
Claudiusweg 7
D-5600 Wuppertal 1(DE)

(72) Erfinder: Lunkenheimer, Winfried, Dr.
Bismarckstrasse 29
D-5600 Wuppertal 1(DE)

(72) Erfinder: Stetter, Jörg, Dr.
Gellertweg 4
D-5600 Wuppertal 1(DE)

(72) Erfinder: Thomas, Rudolf, Dr.
Dellbusch 269
D-5600 Wuppertal 2(DE)

(72) Erfinder: Faust, Wilfried, Dr.
Eifgenstrasse 16
D-5068 Odenthal(DE)

(72) Erfinder: Eue, Ludwig, Dr.
Paul-Klee-Strasse 36
D-5090 Leverkusen(DE)

(54) **Heterocyclisch substituierte Amide, Verfahren zu deren Herstellung und deren Verwendung als Gegenmittel zum Schutz von Kulturpflanzen vor Schädigungen durch Herbizide.**

(57) Heterocyclisch substituierte Amide der Formel

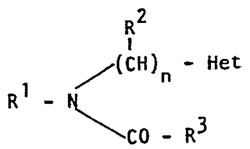

(I)

in welcher

$R^1$ für Alkyl, Alkenyl, Alkinyl, Halogenalkyl, Halogenalkenyl oder Alkoxyalkyl steht,

$R^2$ für Wasserstoff oder Alkyl steht,

$R^3$ für Dihalogenmethyl, Trihalogenmethyl, Halogenalkyl mit mehr als einem Kohlenstoffatom oder für den Rest $-CH_2-O-R^4$ steht,

worin

$R^4$ für Alkylcarbonyl, gegebenenfalls substituiertes Phenylcarbonyl, Alkylsulfonyl, gegebenenfalls substituiertes Phenylsulfonyl, Alkyl, Alkenyl, Alkinyl, Alkoxyalkyl oder Wasserstoff steht,

Het für einen gegebenenfalls substituierten ungesättigten oder mesoionischen Heterocyclus mit 1 bis 3 Heteroatomen steht, wobei als Heteroatome Sauerstoff, Schwefel und Stickstoff in Frage kommen und als Substituenten Alkyl, Alkoxy, Alkylthio, Alkylsulfinyl, Alkylsulfonyl, gegebenenfalls substituiertes Phenyl, Halogen, Hydroxy, Oxo, Mercapto, Thiono, Amino, Imino, Alkylamino und/oder Dialkylamino in Betracht kommen,

und

n für 0, 1 oder 2 steht,

oder

$R^1$ auch für Phenyl steht, wenn $R^2$ für Wasserstoff steht, $R^3$ für Dichlormethyl steht, Het für Fur-2-yl steht und n für 1 steht,

mehrere Verfahren zu deren Herstellung und deren Verwendung als Antidots zum Schutz von Kulturpflanzen vor Schädigungen durch herbizid wirksame Acetanilide und Thiolcarbamate.

Amine der Formel

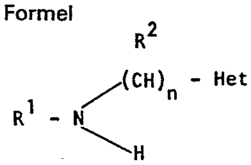

(II)

in welcher

$R^1$, $R^2$, Het und n die oben angegebene Bedeutung haben, und deren Herstellung.

EP 0 049 760 A2

0049760

BAYER AKTIENGESELLSCHAFT 5090 Leverkusen, Bayerwerk
Zentralbereich Dü/mo-C
Patente, Marken und Lizenzen Ib

Heterocyclisch substituierte Amide, Verfahren zu deren Herstellung und deren Verwendung als Gegenmittel zum Schutz von Kulturpflanzen vor Schädigungen durch Herbizide

Die vorliegende Erfindung betrifft neue heterocyclisch substituierte Amide, mehrere Verfahren zu deren Herstellung sowie deren Verwendung als Gegenmittel zum Schutz von Kulturpflanzen vor Schädigungen durch herbizid wirksame Acetanilide und Thiolcarbamate. Ferner betrifft die vorliegende Erfindung neue Wirkstoffkombinationen, die aus neuen heterocyclisch substituierten Amiden und bestimmten herbizid wirksamen Acetaniliden bzw. Thiolcarbamaten bestehen und besonders gute selektive herbizide Eigenschaften besitzen.

Unter "Gegenmitteln" ("Safener", "Antidote") sind im vorliegenden Zusammenhang Stoffe zu verstehen, welche befähigt sind, schädigende Wirkungen von Herbiziden auf Kulturpflanzen spezifisch zu antagonisieren, d.h. die Kulturpflanzen zu schützen, ohne dabei die Herbizidwirkung auf die zu bekämpfenden Unkräuter merklich zu beeinflussen.

Es ist bekannt, daß bestimmte Thiolcarbamate und Acetanilide beim Einsatz zur Unkrautbekämpfung in Mais und anderen Kulturen mehr oder weniger starke Schäden an den Kulturpflanzen hervorrufen. Weiterhin ist bekannt, daß Verbindungen wie z.B. N-Dichloracetyl-2-ethyl-piperidin geeignet sind, um Schädigungen durch Thiolcarbamate oder

Acetanilide an Kulturpflanzen zu vermindern (vgl. DE-OS 22 18 097). Die Wirksamkeit dieses Stoffes als Gegenmittel ist jedoch nicht immer ganz befriedigend.

Es wurden jetzt neue heterocyclisch substituierte Amide der Formel

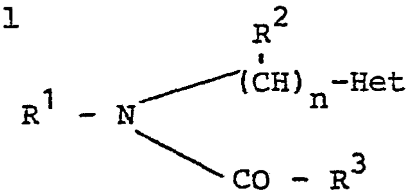

(I)

in welcher

$R^1$ für Alkyl, Alkenyl, Alkinyl, Halogenalkyl, Halogenalkenyl oder Alkoxyalkyl steht,

$R^2$ für Wasserstoff oder Alkyl steht,

$R^3$ für Dihalogenmethyl, Trihalogenmethyl, Halogenalkyl mit mehr als einem Kohlenstoffatom oder für den Rest $-CH_2-O-R^4$ steht,

worin

$R^4$ für Alkylcarbonyl, gegebenenfalls substituiertes Phenylcarbonyl, Alkylsulfonyl, gegebenenfalls substituiertes Phenylsulfonyl, Alkyl, Alkenyl, Alkinyl, Alkoxyalkyl oder Wasserstoff steht,

Het für einen gegebenenfalls substituierten ungesättigten oder mesoionischen Heterocyclus mit 1 bis 3 Heteroatomen steht, wobei als Heteroatome Sauerstoff, Schwefel und Stickstoff in Frage kommen und als Substituenten Alkyl, Alkoxy, Alkylthio, Alkylsulfinyl, Alkylsulfonyl, gegebenenfalls substituiertes Phenyl, Halogen, Hydroxy, Oxo, Mercapto, Thiono, Amino, Imino, Alkylamino und/oder Dialkylamino in Betracht kommen,

und

n für 0, 1 oder 2 steht,

oder

$R^1$ auch für Phenyl steht, wenn $R^2$ für Wasserstoff steht, $R^3$ für Dichlormethyl steht, Het für Fur-2-yl steht und n für 1 steht,

gefunden.

Weiterhin wurde gefunden, daß man heterocyclisch substituierte Amide der Formel (I) erhält, wenn man

a) Amine der Formel

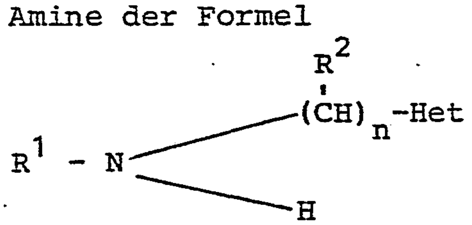

(II)

in welcher
$R^1$, $R^2$, Het und n die oben angegebene Bedeutung haben,

mit Acylhalogeniden der Formel

$R^3 - CO - Y$ (III)

in welcher
Y für Chlor oder Brom steht und
$R^3$ die oben angegebene Bedeutung hat,

in Gegenwart eines Verdünnungsmittels und gegebenenfalls in Gegenwart eines Säurebindemittels umsetzt,

oder

b) Verbindungen der Formel

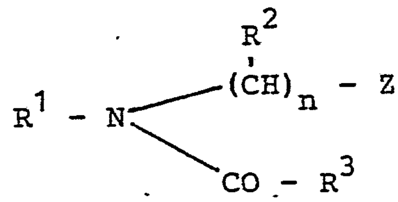

(IV)

in welcher
$R^1$, $R^2$, $R^3$ und n die oben angegebene Bedeutung haben und
Z für eine offenkettige Vorstufe des herzustellenden Heterocyclus steht,

gegebenenfalls in Gegenwart eines Verdünnungsmittels mit cyclisierenden Agenzien behandelt,

oder

c) Verbindungen der Formel

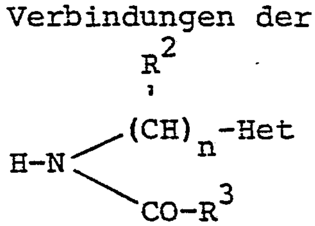

(V)

in welcher
$R^2$, $R^3$, Het und n die oben angegebene Bedeutung haben,

mit Halogenverbindungen der Formel

$R^1 - Y$ (VI)

in welcher

$R^1$ und Y die oben angegebene Bedeutung haben

gegebenenfalls in Gegenwart eines Verdünnungsmittels sowie in Gegenwart eines Säurebindemittels umsetzt,

oder

d) Verbindungen der Formel

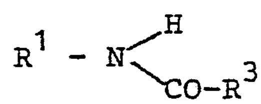

(VII)

in welcher

$R^1$ und $R^3$ die oben angegebene Bedeutung haben,

mit Verbindungen der Formel

$$Y - \overset{R^2}{\overset{|}{(CH)}}_n - Het \qquad \text{(VIII)}$$

in welcher

$R^2$, Y, n und Het die oben angegebene Bedeutung haben,

gegebenenfalls in Gegenwart eines Verdünnungsmittels sowie in Gegenwart eines

Säurebindemittels umsetzt.

Ferner wurde gefunden, daß heterocyclisch substituierte Amide der Formel (I) sich hervorragend verwenden lassen, um Kulturpflanzen vor Schädigungen durch herbizid wirksame Acetanilide bzw. herbizid wirksame Thiolcarbamate zu schützen.

Außerdem wurde gefunden, daß die neuen Wirkstoffkombinationen, bestehend aus einem heterocyclisch substituierten Amid der Formel (I) und mindestens einem herbizid wirksamen Acetanilid bzw. mindestens einem herbizid wirksamen Thiolcarbamat, hervorragend geeignet sind zur selektiven Unkrautbekämpfung in Nutzpflanzenkulturen.

Überraschenderweise werden Herbizidschädigungen durch Acetanilide bzw. durch Thiolcarbamate an Kulturpflanzen bei Mitverwendung von heterocyclisch substituierten Amiden der Formel (I) besser unterdrückt als beim Einsatz des bekannten N-Dichloracetyl-2-ethyl-piperidins, welches ein chemisch ähnlicher Stoff gleicher Wirkungsart ist. Im übrigen war nicht zu erwarten, daß die erfindungsgemäßen Wirkstoffkombinationen bessere selektive herbizide Eigenschaften besitzen als Wirkstoffkombinationen, welche aus mindestens einem herbizid wirksamen Acetanilid bzw. mindestens einem herbizid wirksamen Thiolcarbamat und dem als Gegenmittel bekannten N-Dichloracetyl-2-ethyl-piperidin bestehen.

Die erfindungsgemäßen heterocyclisch substituierten Amide sind durch die Formel (I) allgemein definiert. In dieser Formel steht $R^1$ vorzugsweise für geradkettiges oder verzweigtes Alkyl mit 1 bis 6 Kohlenstoffatomen, Alkenyl mit bis zu 6 Kohlenstoffatomen, Alkinyl mit bis zu 6 Kohlenstoffatomen, Halogenalkyl mit bis zu 6 Kohlenstoffatomen und bis zu 5 Halogenatomen, insbesondere Fluor-, Chlor- und/oder Bromatomen, ferner für Halogenalkenyl mit bis zu 6 Kohlenstoffatomen und bis zu 5 Halogenatomen, insbesondere Fluor-, Chlor- und/oder Bromatomen, sowie Alkoxyalkyl mit 1 bis 6 Kohlenstoffatomen in der Alkylgruppe und 1 bis 6 Kohlenstoffatomen in der Alkoxygruppe. Weiterhin steht $R^1$ auch für Phenyl, wenn $R^2$ für Wasserstoff steht, $R^3$ für Dichlormethyl steht, Het für Fur-2-yl steht und n für 1 steht. $R^2$ steht vorzugsweise für Wasserstoff oder Alkyl mit 1 bis 4 Kohlenstoffatomen. $R^3$ steht vorzugsweise für Dichlormethyl, Dibrommethyl, Trichlormethyl, Tribrommethyl, Halogenalkyl mit 2 bis 6 Kohlenstoffatomen und bis zu 5 Halogenatomen, insbesondere Fluor-, Chlor- und/oder Bromatomen, ferner für den Rest $-CH_2-O-R^4$. Hierbei steht $R^4$ vorzugswiese für Alkylcarbonyl mit bis zu 6 Kohlenstoffatomen, gegebenenfalls durch Alkyl mit 1 bis 4 Kohlenstoffatomen und/oder Halogen substituiertes Phenylcarbonyl, Alkylsulfonyl mit 1 bis 6 Kohlenstoffatomen, gegebenenfalls durch Alkyl mit 1 bis 4 Kohlenstoffatomen und/oder Halogen substituiertes Phenylsulfonyl, Alkyl mit 1 bis 6 Kohlenstoffatomen, Alkenyl mit bis zu 6 Kohlenstoffatomen, Alkinyl mit bis zu 6 Kohlenstoffatomen, Alkoxyalkyl mit 1 bis 6 Kohlenstoffatomen in der Alkylgruppe und 1 bis 6 Kohlenstoffatomen in der Alkoxygruppe sowie für Wasserstoff.
Het steht in der Formel (I) vorzugsweise für die über Stickstoff verknüpften Heterocyclen 1,2,4-Triazolyl, 1,2,3-Triazolyl, Pyrazolyl und Imidazolyl, die jeweils substituiert sein können durch Alkyl mit 1 bis 4 Kohlenstoffatomen, Alkoxy mit 1 bis 4 Kohlenstoffatomen, Alkylthio mit 1 bis 4 Kohlenstoffatomen, Alkylsulfinyl mit 1 bis

4 Kohlenstoffatomen, Alkylsulfonyl mit 1 bis 4 Kohlenstoffatomen, gegebenenfalls durch Halogen und/oder Alkyl mit 1 bis 4 Kohlenstoffatomen substituiertes Phenyl, Fluor, Chlor, Brom, Hydroxy, Mercapto, Amino, Alkylamino mit 1 bis 4 Kohlenstoffatomen und/oder Dialkylamino mit 1 bis 4 Kohlenstoffatomen in jeder Alkylgruppe. Het steht weiterhin für die über Kohlenstoff verknüpften Heterocyclen Pyrimidinyl, Oxazolyl, Isoxazolyl, Thiazolyl, Isothiazolyl, 1,2,4-Oxadiazolyl, 1,3,4-Oxadiazolyl, 1,2,5-Oxadiazolyl, 1,2,4-Thiadiazolyl, 1,3,4-Thiadiazolyl, 1,2,5-Thiadiazolyl, Imidazolyl, Pyrazolyl, 1,2,3-Triazolyl, 1,2,4-Triazolyl und Furyl, die jeweils substituiert sein können durch Alkyl mit 1 bis 4 Kohlenstoffatomen, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, gegebenenfalls durch Halogen und/oder Alkyl mit 1 bis 4 Kohlenstoffatomen substituiertes Phenyl, Fluor, Chlor, Brom, Hydroxy, Oxo, Mercapto, Thiono, Amino, Imino, Alkylamino und Alkylimino mit jeweils 1 bis 4 Kohlenstoffatomen und/oder Dialkylamino mit 1 bis 4 Kohlenstoffatomen in jeder Alkylgruppe.
In der Formel (I) steht n für die Zahlen 0, 1 oder 2.

Besonders bevorzugt sind diejenigen Verbindungen der Formel (I), in denen $R^1$ für Alkyl mit 1 bis 4 Kohlenstoffatomen, Alkenyl mit bis zu 4 Kohlenstoffatomen, Alkinyl mit bis zu 4 Kohlenstoffatomen, Halogenalkyl mit bis zu 4 Kohlenstoffatomen und bis zu 3 Halogenatomen, insbesondere Fluor-, Chlor- und/oder Bromatomen, ferner für Halogenalkenyl mit bis zu 4 Kohlenstoffatomen und bis zu 3 Halogenatomen, insbesondere Fluor-, Chlor- und/oder Bromatomen, sowie Alkoxyalkyl mit 1 bis 4 Kohlenstoffatomen in der Alkylgruppe und 1 bis 4 Kohlenstoffatomen in der Alkoxygruppe steht; beziehungsweise $R^1$ für Phenyl

steht, sofern $R^2$ für Wasserstoff steht, $R^3$ für Dichlormethyl steht, Het für Fur-2-yl steht und n für 1 steht;

$R^2$ für Wasserstoff oder Alkyl mit 1 bis 3 Kohlenstoffatomen steht; $R^3$ für Dichlormethyl, Dibrommethyl, Trichlormethyl, Tribrommethyl, Halogenalkyl mit 2 bis 4 Kohlenstoffatomen und bis zu 3 Fluor-, Chlor- und/oder Bromatomen steht, oder für den Rest $-CH_2-O-R^4$ steht, wobei $R^4$ für Alkylcarbonyl mit bis zu 4 Kohlenstoffatomen, gegebenenfalls durch Alkyl mit 1 bis 3 Kohlenstoffatomen und/oder Chlor substituiertes Phenylcarbonyl, Alkylsulfonyl mit 1 bis 4 Kohlenstoffatomen, gegebenenfalls durch Alkyl mit 1 bis 3 Kohlenstoffatomen und/oder Chlor substituiertes Phenysulfonyl, Alkyl mit 1 bis 4 Kohlenstoffatomen, Alkenyl mit bis zu 4 Kohlenstoffatomen, Alkinyl mit bis zu 4 Kohlenstoffatomen, Alkoxyalkyl mit 1 bis 4 Kohlenstoffatomen in der Alkylgruppe und 1 bis 4 Kohlenstoffatomen in der Alkoxygruppe sowie für Wasserstoff steht; Het für die über Stickstoff verknüpften Heterocyclen 1,2,4-Triazolyl, 1,2,3-Triazolyl, Pyrazolyl und Imidazolyl steht, die jeweils substituiert sein können durch Alkyl mit 1 bis 3 Kohlenstoffatomen, Alkoxy mit 1 bis 3 Kohlenstoffatomen, Alkylthio mit 1 bis 3 Kohlenstoffatomen, Alkylsulfinyl mit 1 bis 3 Kohlenstoffatomen, Alkylsulfonyl mit 1 bis 3 Kohlenstoffatomen, gegebenenfalls durch Chlor und/oder Alkyl mit 1 bis 3 Kohlenstoffatomen substituiertes Phenyl, Fluor, Chlor, Brom, Hydroxy, Mercapto, Amino, Alkylamino mit 1 bis 3 Kohlenstoffatomen und/oder Dialkylamino mit 1 bis 3 Kohlenstoffatomen in jeder Alkylgruppe; und Het weiterhin für die über Kohlenstoff verknüpften Heterocyclen Pyrimidinyl, Oxazolyl, Isoxazolyl, Thiazolyl, Isothiazolyl,

1,2,4-Oxadiazolyl, 1,3,4-Oxadiazolyl, 1,2,5-Oxadiazolyl, 1,2,4-Thiadiazolyl, 1,3,4-Thiadiazolyl, 1,2,5-Thiadiazolyl, Imidazolyl, Pyrazolyl, 1,2,3-Triazolyl, 1,2,4-Triazolyl und Furyl steht, die jeweils substituiert sein können durch Alkyl mit 1 bis 3 Kohlenstoffatomen, Alkoxy mit 1 bis 3 Kohlenstoffatomen, Alkylthio mit 1 bis 3 Kohlenstoffatomen, Alkylsulfinyl mit 1 bis 3 Kohlenstoffatomen, Alkylsulfonyl mit 1 bis 3 Kohlenstoffatomen, gegebenenfalls durch Chlor und/oder Alkyl mit 1 bis 3 Kohlenstoffatomen substituiertes Phenyl, Fluor, Chlor, Brom, Hydroxy, Oxo, Mercapto, Thiono, Amino, Imino, Alkylamino und Alkylimino jeweils 1 bis 3 Kohlenstoffatomen und/oder Dialkylamino mit 1 bis 3 Kohlenstoffatomen in jeder Alkylgruppe; und n für die Zahlen 0, 1 oder 2 steht.

Als Beispiele seien außer den Herstellungsbeispielen die folgenden Verbindungen der allgemeinen Formel (I) genannt:

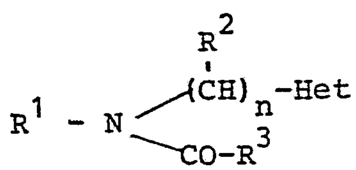

(I)

| $R^1$ | $R^2$ | n | $R^3$ | Het |
|---|---|---|---|---|
| $H_2C=CH-CH_2-$ | H | 1 | $-CHCl_2$ | 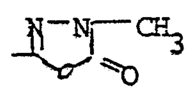 |
| $H_2C=CH-CH_2-$ | H | 1 | $-CHCl_2$ | 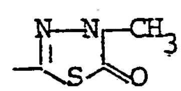 |
| $H_2C=CH-CH_2-$ | H | 1 | $-CHCl_2$ | 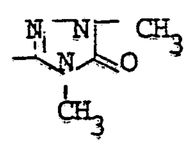 |
| $H_2C=CH-CH_2-$ | H | 1 | $-CHCl_2$ | 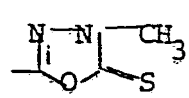 |

| $R^1$ | $R^2$ | n | $R^3$ | Het |
|---|---|---|---|---|
| $H_2C=CH-CH_2-$ | H | 1 | $-CHCl_2$ | 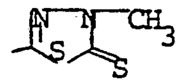 |
| $H_2C=CH-CH_2-$ | H | 1 | $-CHCl_2$ | 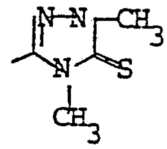 |
| $H_2C=CH-CH_2-$ | H | 1 | $-CHCl_2$ | 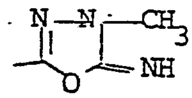 |
| $H_2C=CH-CH_2-$ | H | 1 | $-CHCl_2$ | 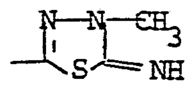 |
| $H_2C=CH-CH_2-$ | H | 1 | $-CHCl_2$ | 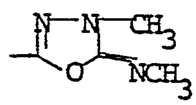 |
| $H_2C=CH-CH_2-$ | H | 1 | $-CHCl_2$ | 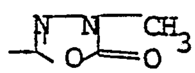 |
| $H_2C=CH-CH_2-$ | H | 1 | $-CHCl_2$ | 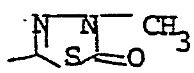 |
| $H_2C=CH-CH_2-$ | H | 1 | $-CHCl_2$ | 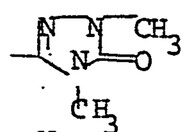 |
| $H_2C=CH-CH_2-$ | H | 1 | $-CHCl_2$ | 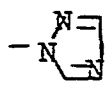 |
| $H_2C=CH-CH_2-$ | H | 1 | $-CHCl_2$ | 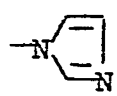 |
| $H_2C=CH-CH_2-$ | H | 1 | $-CH(Cl)-CH_3$ | 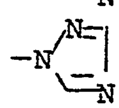 |
| $H_2C=CH-CH_2-$ | H | 1 | $-CH(Cl)-CH_3$ | 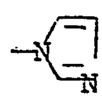 |

Verwendet man N-(5-Ethyl-1,2,4-oxadiazol-3-yl-methyl)-n-butylamin und Dichloracetylchlorid als Ausgangsstoffe, so läßt sich der Verlauf des erfindungsgemäßen Verfahrens (a) durch das folgende Formelschema wiedergeben:

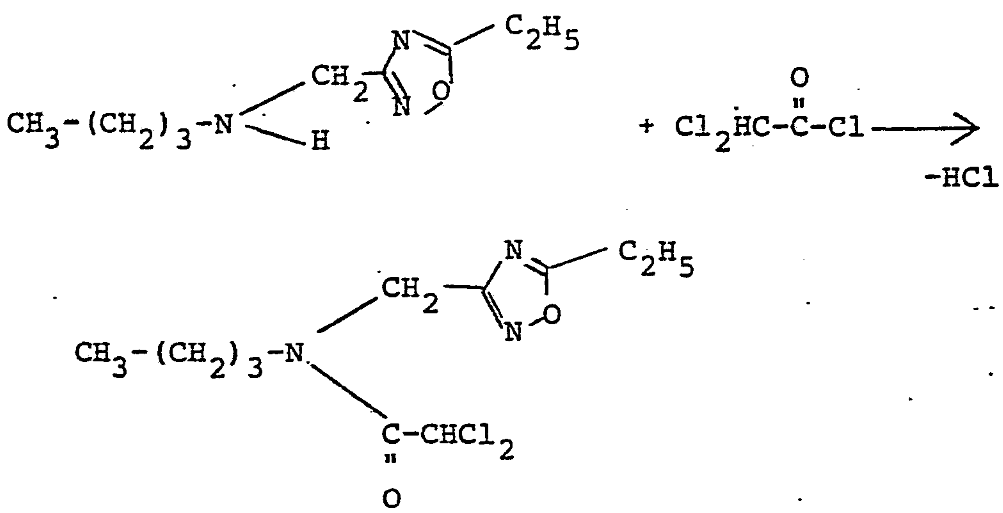


Verwendet man 1-Acetyl-2-/(N-dichlor-acetyl-n-butylamino)-acetyl/-hydrazid als Ausgangsstoff und Schwefelsäure als cyclisierendes Agens, so läßt sich der Verlauf des erfindungsgemäßen Verfahrens (b) durch das folgende Formelschema wiedergeben:

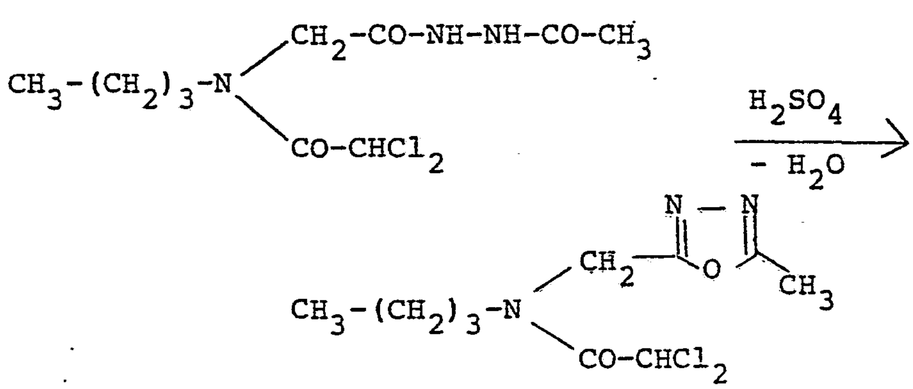

Verwendet man N-(1,2,4-Triazol-1-yl-methyl)-N-dichloracetyl-amin und Allylchlorid als Ausgangsstoffe, so läßt sich der Verlauf des erfindungsgemäßen Verfahrens (c) durch das folgende Formelschema wiedergeben:

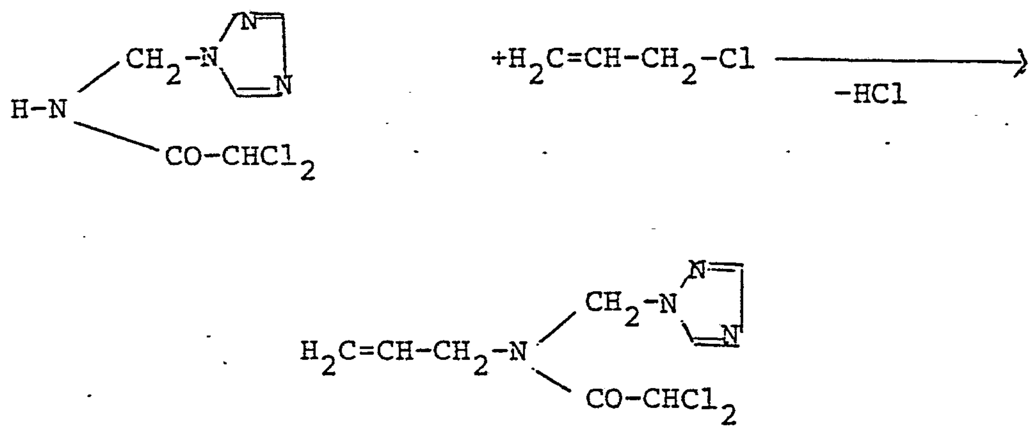


Verwendet man N-Allyl-N-dichloracetylamin und Imidazol-1-yl-methylchlorid als Ausgangsstoffe, so läßt sich der Verlauf des erfindungsgemäßen Verfahrens (d) durch das folgende Formelschema wiedergeben:

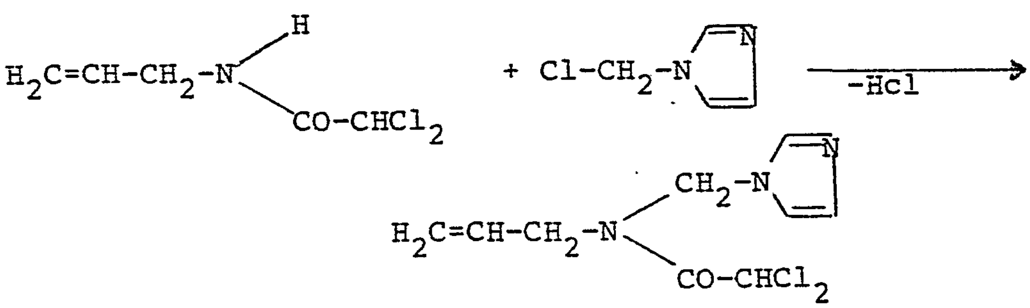

Die bei dem erfindungsgemäßen Verfahren (a) als Ausgangsstoffe benötigten Amine sind durch die Formel (II) allgemein definiert. In dieser Formel haben $R^1$, $R^2$, Het und n vorzugsweise für diejenigen Bedeutungen, die bereits im Zusammenhang mit der Beschreibung der erfindungsgemäßen heterocyclisch substituierten Amide der Formel (I) vorzugsweise für diese Reste bzw. für den Index n genannt wurden.

Die Amine der Formel

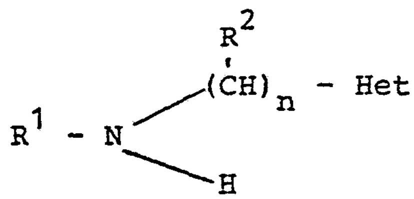
(II)

in welcher

$R^1$ für Alkyl, Alkenyl, Alkinyl, Halogenoalkyl, Halogenalkenyl oder Alkoxyalkyl steht,

$R^2$ für Wasserstoff oder Alkyl steht,

Het für einen gegebenenfalls substituierten ungesättigten oder mesoionischen Heterocyclus mit 1 bis 3 Heteroatomen steht, wobei als Heteroatome Sauerstoff, Schwefel und Stickstoff in Frage kommen und als Substituenten Alkyl, Alkoxy, Alkylthio, Alkylsulfinyl, Alkylsulfonyl, gegebenenfalls substituiertes Phenyl, Halogen, Hydroxy, Oxo, Mercapto, Thiono, Amino, Imino, Alkylamino und/oder Dialkylamino in Betracht kommen,

und

n für 0, 1 oder 2 steht,

oder

$R^1$ auch für Phenyl steht, wenn $R^2$ für Wasserstoff steht, Het für Fur-2-yl steht und n für 1 steht,

sind neu.

Die Amine der Formel (II) lassen sich herstellen, indem man Amine der Formel

$$R^1 - NH_2 \qquad (IX)$$

in welcher
$R^1$ die oben angegebene Bedeutung hat,

mit Verbindungen der Formel

$$Y - (\overset{R^2}{\overset{|}{C}}H)_n - Het \qquad (VIII)$$

in welcher

$R^2$, Y, Het und n die oben angegebene Bedeutung haben,

gegebenenfalls in Gegenwart eines Säurebinders und gegebenenfalls in Gegenwart eines Verdünnungsmittels, oder in einem wässrig-organischen Zweiphasensystem umsetzt.

Die bei der Herstellung der Amine der Formel (II) nach dem obigen Verfahren als Ausgangsstoffe benötigten Amine sind durch die Formel (IX) allgemein definiert. In dieser Formel

steht $R^1$ vorzugsweise für diejenigen Reste, die bereits im Zusammenhang mit der Beschreibung der erfindungsgemäßen heterocyclisch substituierten Amide der Formel (I) vorzugsweise genannt wurden.

Die Amine der Formel (IX) sind bekannt oder lassen sich nach bekannten Verfahren herstellen.

Die bei der Herstellung der Amine der Formel (II) nach dem obigen Verfahren weiterhin als Ausgangsstoffe benötigten Verbindungen sind durch die Formel (VIII) allgemein definiert. In dieser Formel stehen $R^2$, Y, Het und n vorzugsweise für diejenigen Bedeutungen, die bereits im Zusammenhang mit der Beschreibung der erfindungsgemäßen heterocyclisch substituierten Amide der Formel (I) vorzugsweise für $R^2$, Y, Het und n genannt wurden.

Die Verbindungen der Formel (VIII) sind teilweise bekannt (vgl. DE-OS 28 05 757, DE-OS 28 35 156, DE-OS 28 35 157, DE-OS 28 42 280, DE-OS 28 42 284, DE-OS 28 42 315, J. Heterocyclic Chem. 4, 445-446 (1967), J. Am. Chem. Soc. 68, 2393 (1946), Z. Obsch. Khim. 34, 2164 (1964), ibid. 32, 2431 (1961), ibid. 33, 2848 (1963), J. Med. Chem. 7, 808 (1964) und US-PS 3118 889). Die noch nicht bekannten Verbindungen der Formel (VIII) lassen sich nach bekannten Methoden herstellen, indem man zum Beispiel die jeweils zugrunde liegenden, gegebenenfalls substituierten Heterocyclen nach üblichen Methoden am Kern oder in der Seitenkette chloriert oder bromiert.

Die Herstellung der Amine der Formel (II) nach dem oben beschriebenen Verfahren erfolgt gegebenenfalls in Gegenwart eines Säurebindemittels. Als solche können alle üblichen Säurebindemittel verwendet werden. Hierzu gehören vorzugsweise anorganische Basen wie Alkalihydroxide und Alkalicarbonate. Es ist auch möglich das als Ausgangsprodukt dienende Amin der Formel (IX) in einem größeren Überschuß einzusetzen, so daß es gleichzeitig als Reaktionskomponente und als Säurebindemittel fungiert.

Als Verdünnungsmittel kommen bei der Herstellung der Amine der Formel (II) nach dem oben beschriebenen Verfahren Wasser sowie alle inerten, mit Wasser nicht mischbaren, organischen Lösungsmittel in Frage. Hierzu gehören vorzugsweise Ether, wie Diethylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die Reaktionstemperaturen können bei der Herstellung der Amine der Formel (II) nach dem oben beschriebenen Verfahren in einem größeren Bereich variiert werden. Im allgemeinen arbeitet man zwischen -70°C und + 100°C, vorzugsweise zwischen -20°C und + 80°C.

Bei der Durchführung des Verfahrens zur Herstellung der Amine der Formel (II) setzt man auf 1 Mol einer Verbindung der Formel (VIII) 1 bis 3 Mol an Amin der Formel (IX) sowie 1 bis 4 Mol an Säurebindemittel ein. Die Isolierung der Amine der Formel (II) erfolgt in üblicher Weise.

Die Verbindung der Formel

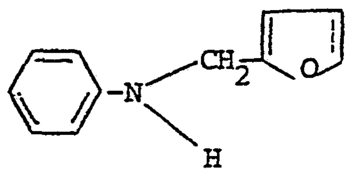

die von der allgemeinen Formel (II) umfaßt wird, läßt sich auch dadurch herstellen, daß man Anilin mit Furfural umsetzt und die dabei entstehende Schiff'sche Base mit einem Reduktionsmittel, wie Natriumborhydrid, behandelt. Formelmäßig läßt sich der Verlauf dieses Verfahrens wie folgt darstellen:

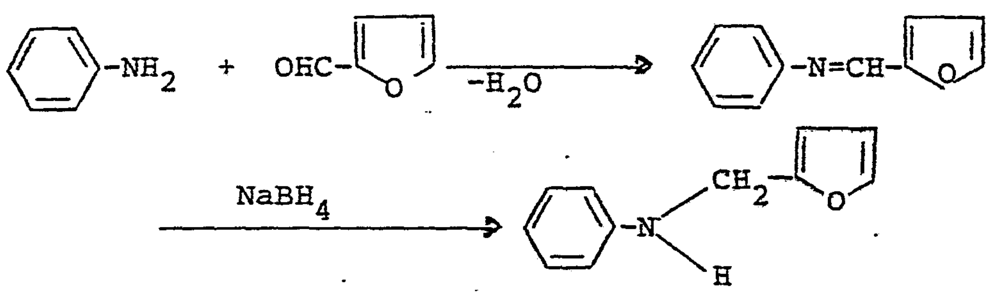


Die bei dem erfindungsgemäßen Verfahren (a) weiterhin als Ausgangsstoffe benötigten Acylhalogenide sind durch die Formel (III) allgemein definiert. In dieser Formel steht $R^3$ vorzugsweise für diejenigen Reste, die bereits im Zusammenhang mit der Beschreibung der erfindungsgemäßen heterocyclisch substituierten Amide der Formel (I) vorzugsweise für $R^3$ genannt wurden. Y steht für Chlor oder Brom. Die Acylhalogenide der Formel (III) sind bekannt oder lassen sich nach bekannten Verfahren herstellen.

Als Verdünnungsmittel kommen für die erfindungsgemäße Umsetzung gemäß Verfahrensvariante (a) vorzugsweise inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol, halogenierte Kohlenwasserstoffe, wie Methylenchlorid, Tetrachlorkohlenstoff, Chloroform oder Chlorbenzol; und Ester, wie Essigester.

Das erfindungsgemäße Verfahren (a) kann gegebenenfalls in Gegenwart von Säurebindern durchgeführt werden. Als solche können alle üblichen Säurebindemittel verwendet werden. Hierzu gehören vorzugsweise organische Basen, wie tertiäre Amine, beispielsweise Triethylamin, oder wie Pyridin, ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen 0°C und 120°C, vorzugsweise zwischen 20°C und 100°C.

Bei der Durchführung des erfindungsgemäßen Verfahrens (a) setzt man vorzugsweise auf 1 Mol Amin der Formel (II) 1 bis 1,5 Mol an Acylhalogenid der Formel (III) und gegebenenfalls 1,0 bis 1,5 Mol Säurebinder ein. Die Isolierung der Verbindungen der Formel (I) erfolgt in üblicher Weise.

Nach einer speziellen Variante des erfindungsgemäßen Verfahrens (a) erhält man solche Verbindungen der Formel (I), in denen Het für Hydroxyheterocyclen bzw. deren tautomere Oxoformen steht, indem man solche Amine der Formel (II), in welchen Het für die entsprechenden durch Alkoxy substituierten Heterocyclen steht, mit Acylhalogeniden der Formel (VII) in Gegenwart eines Verdünnungsmittels und gegebenenfalls in Gegenwart eines Katalysators, wie zum Beispiel Dimethylformamid, in Abwesenheit eines Säurebindemittels umsetzt. Bei der Durchführung dieser Variante des Verfahrens (a) wird Alkylhalogenid abgespalten.

Die bei der Durchführung des erfindungsgemäßen Verfahrens (b) als Ausgangsstoffe benötigten Verbindungen sind durch die Formel (IV) allgemein definiert. In dieser Formel stehen $R^1$, $R^2$, $R^3$ und n vorzugsweise für diejenigen Bedeutungen, die bereits im Zusammenhang mit der Beschreibung der erfindungsgemäßen heterocyclisch substituierten Amide der Formel (I) vorzugsweise für $R^1$, $R^2$, $R^3$ und n genannt wurden. Z steht für eine offenkettige Vorstufe des jeweils herzustellenden Heterocyclus. So kann Z beispielsweise für den Rest der Formel $-CO-NH-NH-\underset{\overset{\|}{X}}{C}-R^5$

stehen, worin
X für Sauerstoff oder Schwefel steht und $R^5$ für Alkyl oder gegebenenfalls durch Halogen und/oder Alkyl substituiertes Phenyl steht. Vorzugsweise steht $R^5$ hierbei für Alkyl mit 1 bis 4 Kohlenstoffatomen oder für gegebenenfalls durch Chlor und/oder Alkyl mit 1 bis 4 Kohlenstoffatomen substituiertes Phenyl.

Die Verbindungen der Formel (IV) sind bekannt oder lassen sich nach im Prinzip bekannten Verfahren herstellen.

Als cyclisierende Agenzien kommen bei dem erfindungsgemäßen Verfahren (b) alle für derartige Ringschlußreaktionen geeigneten Stoffe in Betracht. Hierzu gehören zum Beispiel wasserabspaltende Stoffe, wie Schwefelsäure, Phosphoroxychlorid und andere saure Agenzien.

Bei dem erfindungsgemäßen Verfahren (b) kann gegebenenfalls in einem inerten Verdünnungsmittel gearbeitet werden. Die Umsetzungen nach dem erfindungsgemäßen Verfahren (b) erfolgen in an sich bekannter Weise (vgl. Chem. Ber. 32, 797 (1899), J. prakt. Chemie 69, 145 (1904) und Elderfield, "Heterocyclic Compounds", Vol. 7, (1961)).

Die bei der Durchführung des erfindungsgemäßen Verfahrens (c) als Ausgangsstoffe benötigten Verbindungen sind durch die Formel (V) allgemein definiert. In dieser Formel haben $R^2$, $R^3$, Het und n vorzugsweise diejenigen Bedeutungen, die bereits im Zusammenhang mit der Beschreibung der erfindungsgemäßen heterocyclisch substituierten Amide der Formel (I) vorzugsweise für diese Reste beziehungsweise für n genannt wurden.

Die Verbindung der Formel

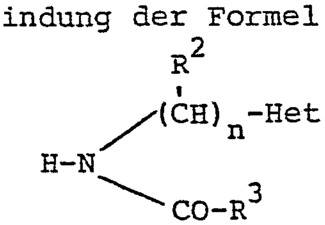

(V)

in welcher

$R^2$ für Wasserstoff oder Alkyl steht,

$R^3$ für Dihalogenmethyl, Trihalogenmethyl, Halogenalkyl mit mehr als einem Kohlenstoffatom oder für den Rest $-CH_2-O-R^4$ steht,

worin

$R^4$ für Alkylcarbonyl, gegebenenfalls substituiertes Phenylcarbonyl, Alkylsulfonyl, gegebenenfalls substituiertes Phenylsulfonyl, Alkyl, Alkenyl, Alkinyl, Alkoxyalkyl oder Wasserstoff steht,

Het für einen gegebenenfalls substituierten ungesättigten oder mesoionischen Heterocyclus mit 1 bis 3 Heteroatomen steht, wobei als Heteroatome Sauerstoff, Schwefel und Stickstoff in Frage kommen und als Substituenten Alkyl, Alkoxy, Alkylthio, Alkylsulfinyl, Alkylsulfonyl, gegebenenfalls substituiertes Phenyl, Halogen, Hydroxy, Oxo, Mercapto, Thiono, Amino, Imino, Alkylamino und/oder Dialkylamino in Betracht kommen,

und

n für 0, 1 oder 2 steht,

sind bisher noch nicht bekannt.

Die Verbindungen der Formel (V) lassen sich herstellen, indem man Amin-Derivate der Formel

$$H_2N-(\overset{R^2}{\overset{|}{C}}H)_n-Het \qquad (X)$$

in welcher

$R^2$, Het und n die oben angegebene Bedeutung haben,

mit Acylhalogeniden der Formel

$R^3$-CO-Y (III)

in welcher

$R^3$ und Y die oben angegebene Bedeutung haben,

in Gegenwart eines Verdünnungsmittels sowie gegebenenfalls in Gegenwart eines Säurebindemittels umsetzt.

Die Verbindungen der Formel (X) sind bekannt oder lassen sich nach im Prinzip bekannten Verfahren herstellen.

Die Herstellung der Verbindungen der Formel (V) erfolgt bevorzugt in Gegenwart eines Säurebindemittels. Als solche können alle üblichen Säureakzeptoren verwendet werden. Hierzu gehören vorzugsweise diejenigen Säurebindemittel, die bereits im Zusammenhang mit dem Verfahren zur Herstellung der Amine der Formel (II) als Säureakzeptoren genannt wurden.

Als Verdünnungsmittel kommen bei der Herstellung der Verbindungen der Formel (V) nach dem oben beschriebenen Verfahren alle üblichen inerten Solventien in Frage.

Die Reaktionstemperaturen können bei der Herstellung der Verbindungen der Formel (V) nach dem oben beschriebenen Verfahren in einem größeren Bereich variiert werden. Im allgemeinen arbeitet man bei Temperaturen zwischen -70°C und + 100°C, vorzugsweise zwischen -20°C und +80°C.

Die Durchführung des oben beschriebenen Verfahrens zur Herstellung der Verbindungen der Formel (V) sowie die Isolierung der Stoffe der Formel (V) erfolgen in üblicher Weise.

Die bei der Durchführung des erfindungsgemäßen Verfahrens (c) weiterhin als Ausgangsstoffe benötigten Halogenverbindungen sind durch die Formel (VI) allgemein definiert. In dieser Formel steht $R^1$ vorzugsweise für diejenigen Reste, die bereits im Zusammenhang mit der Beschreibung der erfindungsgemäßen heterocyclisch substituierten Amide der Formel (I) vorzugsweise für $R^1$ genannt wurden. Y steht für Chlor oder Brom.

Die Halogenverbindungen der Formel (VI) sind bekannt oder lassen sich nach im Prinzip bekannten Methoden herstellen.

Die bei der Durchführung des erfindungsgemäßen Verfahrens (d) als Ausgangsstoffe benötigten Verbindungen sind durch die Formel (VII) allgemein definiert. In dieser Formel haben $R^1$ und $R^3$ vorzugsweise diejenigen Bedeutungen, die bereits im Zusammenhang mit der Beschreibung der erfindungsgemäßen

Stoffe der Formel (I) vorzugsweise für diese Reste genannt wurden.

Die Verbindungen der Formel (VII) sind bekannt oder lassen sich nach im Prinzip bekannten Verfahren herstellen.

Die bei der Durchführung des Verfahrens (d) weiterhin als Ausgangsstoffe benötigten Verbindungen der Formel (VIII) wurden bereits im Zusammenhang mit dem Verfahren zur Herstellung der Amine der Formel (II) beschrieben.

Die erfindungsgemäßen Verfahren (c) und (d) werden in Gegenwart von Säurebindemitteln durchgeführt. Als solche können alle üblichen Säureakzeptoren verwendet werden. Hierzu gehören vorzugsweise organische Basen, wie tertiäre Amine, beispielsweise Triethylamin oder Pyridin, ferner anorganische Basen, wie beispielsweise Alkalihydroxide und Alkalicarbonate.

Als Verdünnungsmittel kommen bei der Durchführung der erfindungsgemäßen Verfahren (c) und (d) alle inerten organischen Solventien in Frage. Hierzu gehören vorzugsweise alle diejenigen Lösungsmittel, die bereits im Zusammenhang mit der Beschreibung des erfindungsgemäßen Verfahrens (a) als bevorzugt genannt wurden.

Die Reaktionstemperaturen können bei der Durchführung der erfindungsgemäßen Verfahren (c) und (d)

in einem größeren Bereich variiert werden. Im allgemeinen arbeitet man bei Temperaturen zwischen 0°C und 120°C, vorzugsweise zwischen 20°C und 100°C.

Die praktische Durchführung und die Aufarbeitung erfolgen bei den erfindungsgemäßen Verfahren (c) und (d) nach üblichen Methoden. Im einzelnen verfährt man so, wie es bereits im Zusammenhang mit dem erfindungsgemäßen Verfahren (a) beschrieben wurde.

Die erfindungsgemäßen heterocyclisch substituierten Amide der Formel (I) eignen sich - wie bereits erwähnt - zum Schutz von Kulturpflanzen vor Schädigungen durch herbizid wirksame Thiolcarbamate und Acetanilide, ohne deren Unkrautwirkung merklich zu beeinflussen.

Vorzugsweise können die erfindungsgemäßen heterocyclisch substituierten Amide der Formel (I) als Gegenmittel zum Schutz von Kulturpflanzen vor Schädigungen durch herbizid wirksame Thiolcarbamate der Formel

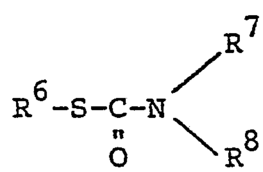

(XI)

in welcher

$R^6$ für niederes Alkyl, Benzyl, Chlorbenzyl oder Alkoxybenzyl steht,

$R^7$ und $R^8$ unabhängig voneinander für Alkyl mit 2 bis 4 Kohlenstoffatomen oder für Cyclohexyl stehen und außerdem

$R^7$ und $R^8$ gemeinsam mit dem angrenzenden Stickstoffatom für einen fünf- bis siebengliedrigen heterocyclischen Ring stehen,

bzw. zum Schutz von Kulturpflanzen vor Schädigungen durch herbizid wirksame Acetanilide der Formel

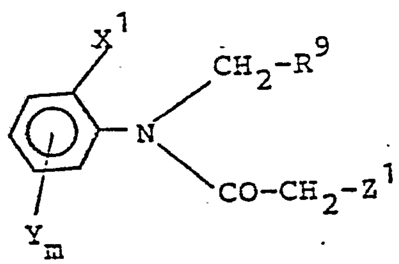

(XII)

in welcher

$R^9$ für einen gegebenenfalls substituierten N-haltigen heterocyclischen Rest steht,

$X^1$ und $Y^1$ gleich oder verschieden sind und für Alkyl stehen,

$Z^1$ für Halogen steht und

m für 0, 1 oder 2 steht,

sowie deren herbizid-wirksame Säureadditionssalze und Metallsalz-Komplexe,

bzw. der Formel

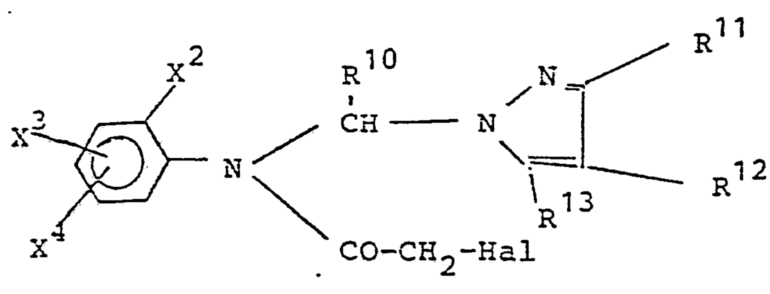

(XIII)

in welcher

Hal für Halogen steht,

$R^{10}$ für Alkyl, Cycloalkyl, Halogenalkyl, Alkoxyalkyl, Alkenyl, Alkinyl oder gegebenenfalls substituiertes Phenyl steht,

$R^{11}$, $R^{12}$ und $R^{13}$ gleich oder verschieden sind und für Wasserstoff, Alkyl, Halogen oder Alkoxy stehen, und

$X^2$, $X^3$ und $X^4$ gleich oder verschieden sind und für Wasserstoff oder Alkyl stehen,

sowie deren herbizid wirksame Säureadditions-Salze und Metallsalz-Komplexe,

- bzw. der Formel

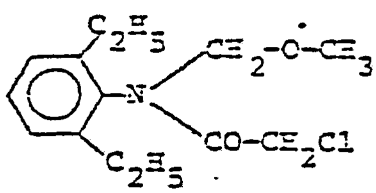

(XIV)

- oder der Formel

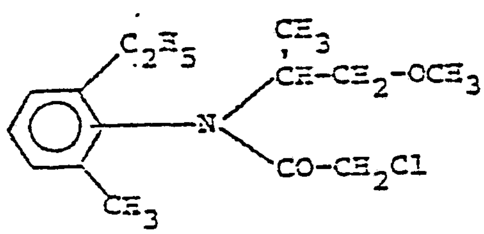

(XV)

verwendet werden.

Weiterhin können die erfindungsgemäßen heterocyclisch substiuierten Amide der Formel (I) vorzugsweise als Gegenmittel zum Schutz von Kulturpflanzen vor Schädigungen durch herbizid wirksame substituierte α-Halogenacetanilide, die aus der DE-OS 29 18 297 bekannt sind, verwendet werden.

Als Beispiele für Thiolcarbamate der Formel (XI)

seien im einzelnen genannt:

S-Ethyl-N,N-dipropylthiocarbamat
S-Ethyl-N,N-diisobutylthiocarbamat
S-Propyl-N-butyl-N-ethylthiocarbamat
S-Propyl-N,N-diisopropylthiocarbamat
S-Ethyl-N,N-diethylthiocarbamat
S-Ethyl-N-ethyl-N-cyclohexylthiocarbamat
S-Ethyl-hexahydro-azepin-1-thiocarbamat
S-p-Methoxybenzyl-N,N-diethylthiocarbamat
S-p-Chlorbenzyl-N,N-diethylthiocarbamat
S-Benzyl-N,N-diethylthiocarbamat
S-Benzyl-N,N-di-sek.-butylthiocarbamat
S-Propyl-N-ethyl-N-butylthiocarbamat

Die Thiolcarbamate der Formel (XI) und deren herbizide Wirksamkeit sind bereits bekannt (vgl. US-Patentschriften 2 913 327, 3 037 853, 3 185 720, 3 198 786 und 3 582 314).

In der Formel (XII) steht $R^9$ vorzugsweise für die über N gebundenen, gegebenenfalls substituierten Azolylreste Pyrazol-1-yl, Imidazol-1-yl, 1,2,4-Triazol-1-yl; 1,2,3-Triazol-1-yl; 1,3,4-Triazol-1-yl und 1,2,3,4,-Tetrazol-1-yl sowie für gegebenenfalls substituiertes Pyrrol-1-yl. Als Substituenten kommen vorzugsweise in Frage: Halogen, insbesondere Fluor, Chlor und Brom, sowie Alkyl mit 1 bis 4 Kohlenstoffatomen. $X^1$ und $Y^1$ sind gleich oder verschieden und stehen vorzugsweise für geradkettiges oder verzweigtes Alkyl mit 1 bis 4 Kohlenstoffatomen. $Z^1$ steht vorzugsweise für die Halogene Chlor und Brom und der Index m steht für O, 1 oder 2.

Als Beispiele für Acetanilide der Formel (XII) seien im einzelnen genannt:

2-Methyl-6-ethyl-N-(pyrazol-1-yl-methyl)-chloracetanilid

2,6-Diethyl-N-(pyrazol-1-yl-methyl)-chloracetanilid

2,6-Diethyl-N-(1,2,4-triazol-1-yl-methyl)-chloracetanilid

2,6-Dimethyl-N-(1,2,4-triazol-1-yl-methyl)-chloracetanilid

2-Methyl-N-(pyrazol-1-yl-methyl)-chloracetanilid

2,5-Dimethyl-N-(pyrazol-1-yl-methyl)-chloracetanilid

2,3-Dimethyl-N-(pyrazol-1-yl-methyl)-chloracetanilid

2-Methyl-6-ethyl-N-(pyrazol-1-yl-methyl)-chloracetanilid-hydrochlorid

2,6-Diethyl-N-(pyrazol-1-yl-methyl)-chloracetanilid-hydrochlorid

2,6-Diethyl-N-[(3,5-dimethyl-pyrazol-1-yl)-methyl]-chloracetanilid

2,6-Diethyl-N-[(3-chlor-1,2,4-triazol-1-yl)-methyl]-chloracetanilid

2-Methyl-6-ethyl-N-[(3,5-dimethyl-pyrazol-1-yl)-methyl]-chloracetanilid

2-tert.-Butyl-N-(pyrazol-1-yl-methyl)-chloracetanilid

2-Methyl-6-ethyl-N-[(3-brom-5-methyl-pyrazol-1-yl)-methyl]-chloracetanilid

2-Methyl-6-ethyl-N-[(3-chlor-1,2,4-triazol-1-yl)-methyl]-chloracetanilid

2,6-Diethyl-N-[(4-Chlor-pyrazol-1-yl)-methyl]-chloracetanilid

Weitere bevorzugt in Frage kommende Acetanilide der Formel (XII) sind in der nachfolgenden Tabelle 1 formelmäßig aufgeführt.

Tabelle 1

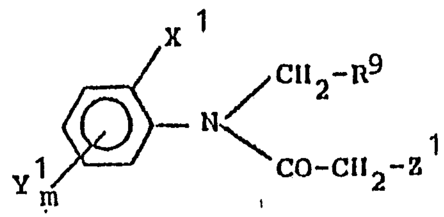

(XII)

| Bsp.-Nr. | $X^1$ | $Y^1_m$ | $Z^1$ | $R^9$ |
|---|---|---|---|---|
| XII -1 | $C_2H_5$ | $6-C_2H_5$ | Cl | Pyrazol-1-yl |
| " -2 | $C_2H_5$ | $6-C_2H_5$ | Cl | 1,2,4-Triazol-1-yl |
| " -3 | $i-C_3H_7$ | $6-i-C_3H_7$ | Cl | Pyrazol-1-yl |
| " -4 | $CH_3$ | $6-C_2H_5$ | Cl | 1,2,4-Triazol-1-yl |
| " -5 | $CH_3$ | $6-C_2H_5$ | Cl | Pyrazol-1-yl |
| " -6 | $C_2H_5$ | $4,6-(CH_3)_2$ | Cl | Pyrazol-1-yl |
| " -7 | $CH_3$ | $4,6-(CH_3)_2$ | Cl | Pyrazol-1-yl |
| " -8 | $C_2H_5$ | $4-CH_3$, $6-C_2H_5$ | Cl | Pyrazol-1-yl |
| " | | | | |
| " -9 | $i-C_3H_7$ | $6-i-C_3H_7$ | Cl | 1,3,4-Triazol-1-yl |
| " -10 | $i-C_3H_7$ | $6-i-C_3H_7$ | Cl | 1,2,4-Triazol-1-yl |
| " -11 | $C_2H_5$ | $6-C_2H_5$ | Cl | Pyrrol-1-yl |
| " -12 | $i-C_3H_7$ | - | Cl | 1,2,4-Triazol-1-yl |
| " -13 | $CH_3$ | $6-C_2H_5$ | Cl | 1,2,3,4-Tetrazol-1-yl |
| " -14 | $i-C_3H_7$ | - | Cl | Pyrazol-1-yl |
| " -15 | $C_2H_5$ | - | Cl | 1,2,4-Triazol-1-yl |

Tabelle 1 (Fortsetzung)

| Beispiel Nr. | $X^1$ | $Y^1_m$ | $Z^1$ | $R^9$ |
|---|---|---|---|---|
| XII -16 | $CH_3$ | 6-$CH_3$ | Cl | Pyrazol-1-yl |
| " -17 | $CH_3$ | 6-$CH_3$ | Cl | 1,2,4-Triazol-1-yl |
| " -18 | $CH_3$ | 5-$CH_3$ | Cl | 1,2,4-Triazol-1-yl |
| " -19 | $CH_3$ | - | Cl | Pyrazol-1-yl |
| " -20 | $CH_3$ | - | Cl | 1,2,4-Triazol-1-yl |
| " -21 | $CH_3$ | 5-$CH_3$ | Cl | Pyrazol-1-yl |
| " -22 | $CH_3$ | 3-$CH_3$ | Cl | 1,2,4-Triazol-1-yl |
| " -23 | $CH_3$ | 3-$CH_3$ | Cl | Pyrazol-1-yl |
| " -24 | $C_2H_5$ | 6-$CH_3$ | Cl | Pyrazol-1-yl (xHCl) |
| " -25 | $C_2H_5$ | 6-$C_2H_5$ | Cl | Pyrazol-1-yl (xHCl) |
| " -26 | $C_2H_5$ | 6-$C_2H_5$ | Cl | 3,5-Dimethyl-pyrazol-1-yl |
| " -27 | $C_2H_5$ | 6-$C_2H_5$ | Cl | Brom-methyl-pyrazolyl |
| " -28 | $C_2H_5$ | 6-$C_2H_5$ | Cl | 3-Chlor-1,2,4-triazol-1-yl |

Tabelle 1 (Fortsetzung)

| Beispiel Nr. | $X^1$ | $Y^1_m$ | $Z^1$ | $R^9$ |
|---|---|---|---|---|
| XII -29 | $CH_3$ | 6-$C_2H_5$ | Cl | 3,5-Dimethyl-pyrazol-1-yl |
| " -30 | $C_2H_5$ | 6-$C_2H_5$ | Cl | 3-Methyl-pyrazol-1-yl |
| " -31 | $C_2H_5$ | 6-$CH_3$ | Cl | 3-Methyl-pyrazol-1-yl |
| " -32 | $C(CH_3)_3$ | - | Cl | Pyrazol-1-yl |
| " -33 | $C(CH_3)_3$ | - | Cl | 1,2,4-Triazol-1-yl |
| " -34 | $C_2H_5$ | 6-$CH_3$ | Cl | Brom-methyl-pyrazolyl |
| " -35 | $CH_3$ | 6-$C_2H_5$ | Cl | 4-Chlor-pyrazol-1-yl |
| " -36 | $CH_3$ | 6-$C_2H_5$ | Cl | 3-Chlor-1,2,4-triazol-1-yl |
| " -37 | $C_2H_5$ | 6-$CH_3$ | Cl | 2,4,5-Trichlor-imidazol-1-yl |
| " -38 | $C_2H_5$ | 6-$C_2H_5$ | Cl | 4-Chlor-pyrazol-1-yl |
| " -39 | $C_2H_5$ | 6-$C_2H_5$ | Cl | 1,2,3,4-Tetrazol-1-yl |

Tabelle 1 (Fortsetzung)

| Beispiel Nr. | $X^1$ | $Y^1_m$ | $Z^1$ | $R^9$ |
|---|---|---|---|---|
| XII -40 | $C_2H_5$ | $6-C_2H_5$ | Br | Pyrazol-1-yl |
| " -41 | $CH_3$ | $6-C_2H_5$ | Br | Pyrazol-1-yl |
| " -42 | $C_2H_5$ | $6-C_2H_5$ | Cl | Imidazol-1-yl |
| " -43 | $C_2H_5$ | $6-C_2H_5$ | Br | 1,2,4-Triazol-1-yl |
| " -44 | $CH_3$ | $6-C_2H_5$ | Br | 1,2,4-Triazol-1-yl |

Die Acetanilide der Formel (XII) und deren herbizide Wirksamkeit sowie deren herbizid wirksame Säureadditionssalze und Metallsalz-Komplexe sind bereits bekannt (vgl. DE-OS 2 648 008 und DE-OS 2 704 281).

In der Formel (XIII) steht Hal vorzugsweise für die Halogene Fluor, Chlor und Brom. $R^{10}$ steht vorzugsweise für geradkettiges oder verzweigtes Alkyl mit 1 bis 8 Kohlenstoffatomen, Cycloalkyl mit 3 bis 6 Kohlenstoffatomen, Halogenalkyl mit bis zu 2 Kohlenstoff- und bis zu 3 gleichen oder verschiedenen Halogenatomen, wobei als Halogenatome vorzugsweise Fluor und Chlor genannt seien, Alkoxyalkyl mit 1 bis 4 Kohlenstoffatomen im Alkylteil und 1 bis 4 Kohlenstoffatomen im Alkoxyteil, ferner für Alkenyl und Alkinyl mit jeweils 2 bis 4 Kohlenstoffatomen sowie für gegebenenfalls substituiertes Phenyl, wobei als Substituenten vorzugsweise in Frage kommen: Halogen, Alkyl mit 1 bis 4 Kohlenstoffatomen, Halogenalkyl mit bis zu 2 Kohlenstoff- und bis zu 3 gleichen oder verschiedenen Halogenatomen, wobei als Halogene vorzugsweise Fluor und Chlor genannt seien, Alkoxy und Alkylthio mit jeweils bis zu 2 Kohlenstoffatomen, Cyano und Nitro. $R^{11}$, $R^{12}$ und $R^{13}$ sind gleich oder verschieden und stehen vorzugsweise für Wasserstoff, geradkettiges oder verzweigtes Alkyl mit 1 bis 4 Kohlenstoffatomen, Halogen und Alkoxy mit 1 bis 4 Kohlenstoffatomen. $X^2$, $X^3$ und $X^4$ sind gleich oder verschieden und stehen vorzugsweise für Wasserstoff und geradkettiges oder verzweigtes Alkyl mit 1 bis 4 Kohlenstoffatomen.

Weitere bevorzugt in Frage kommende Acetanilide der Formel (XIII) sind in der nachfolgenden Tabelle 2 formelmäßig aufgeführt.

Tabelle 2:

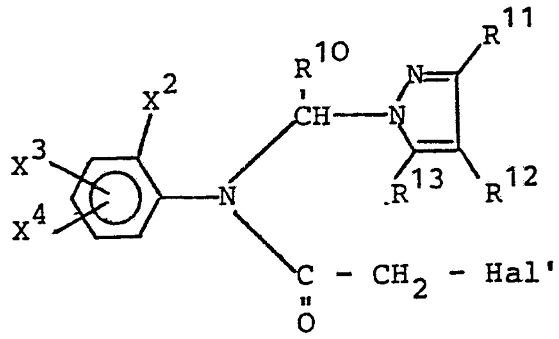

(XIII)

| Beispiel Nr. | $X^2$ | $X^3$ | $X^4$ | $R^{10}$ | $R^{11}$ | $R^{12}$ | $R^{13}$ | Hal' |
|---|---|---|---|---|---|---|---|---|
| XIII-1 | $CH_3$ | $6-C_2H_5$ | H | $CH_3$ | H | H | H | Cl |
| XIII-2 | $CH_3$ | $6-CH_3$ | H | $CH_3$ | H | H | H | Cl |
| XIII-3 | $C_2H_5$ | $6-C_2H_5$ | H | $CH_3$ | H | H | H | Cl |

Die Acetanilide der Formel (XIII) und deren herbizide Wirksamkeit sowie deren herbizid wirksame Säureadditions-Salze und Metallsalz-Komplexe sind bereits bekannt (vgl. DE-OS 28 35 156).

Weitere bevorzugt in Frage kommende Acetanilide, bei denen die erfindungsgemäßen Verbindungen der Formel (I) als Gegenmittel eingesetzt werden können, sind die Verbindungen der Formel (XIV) und (XV). Diese Stoffe und deren herbizide Wirksamkeit sind bereits bekannt (vgl. US-PS 3 442 945, DE-OS 2 328 340).

Die erfindungsgemäßen heterocyclisch substituierten Amide der Formel (I) eignen sich insbesondere zum Schutz von wichtigen Kulturpflanzen, wie Mais, Sojabohnen, Baumwolle, Zuckerrüben, Getreide, Reis und Zuckerrohr vor Herbizidschäden durch Acetanilide und Thiolcarbamate.

Die erfindungsgemäßen Wirkstoffkombinationen bestehend aus einem heterocyclisch substituierten Amid der Formel (I) und mindestens einem herbizid wirksamen Acetanilid bzw. mindestens einem herbizid wirksamen Thiolcarbamat zeigen eine sehr gute Wirkung gegen Unkräuter und Ungräser in zahlreichen Nutzpflanzenkulturen. Sie können daher zur selektiven Unkrautbekämpfung in zahlreichen Nutzpflanzenkulturen verwendet werden. - Unter Unkräutern im weitesten Sinne sind hierbei alle Pflanzen zu verstehen, die an Orten wachsen, wo sie unerwünscht sind.

Die erfindungsgemäßen Wirkstoffkombinationen können z.B. bei folgenden Pflanzen verwendet werden.

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca; Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Insbesondere eignen sich die erfindungsgemäßen Wirkstoffkombinationen zur selektiven Unkrautbekämpfung in Mais, Sojabohnen, Baumwolle, Zuckerrüben, Getreide, Reis und Zuckerrohr.

Die erfindungsgemäßen Gegenmittel können gegebenenfalls im Gemisch mit den herbiziden Wirkstoffen, bei denen sie eingesetzt werden, in die üblichen Formulierungen übergeführt werden, wie Lösungen, Emulsionen, Spritzpulver, Suspensionen, Pulver, Stäubemittel, Schäume, lösliche Pulver, Granulate, Suspensions-Emulsionskonzentrate, wirkstoffimprägnierte Natur- und synthetische Stoffe, Feinstverkapselungen in polymeren Stoffen und in Hüllmassen für Saatgut.

Diese Formulierungen werden in bekannter Weise hergestellt, z.B. durch Vermischen der erfindungsgemäßen Gegenmittel gegebenenfalls im Gemisch mit den herbiziden Wirkstoffen, bei denen sie eingesetzt werden, mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% an Gegenmittel bzw. an Gegenmittel und herbizidem Wirkstoff, vorzugsweise zwischen 0,5 und 90 %.

Die erfindungsgemäßen Gegenmittel können als solche oder in ihren Formulierungen auch in Mischung mit herbiziden Wirkstoffen eingesetzt werden, wobei Fertigformulierungen oder Tankmischung möglich ist. Auch eine Mischung mit anderen bekannten Wirkstoffen wie Fungiziden, Insektiziden, Akariziden, Nematiziden, Schutzstoffen gegen Vogelfraß, Wuchstoffen, Pflanzennährstoffen und Bodenstrukturverbesserungsmitteln ist möglich.

Die erfindungsgemäßen Gegenmittel bzw. Gemische aus erfindungsgemäßen Gegenmitteln und herbizidem Wirkstoff können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Gießen, Spritzen, Sprühen, Stäuben oder Streuen, Trockenbeizen, Feuchtbeizen, Naßbeizen, Schlämmbeizen oder Inkrustieren.

Die erfindungsgemäßen Gegenmittel können nach den für derartige Antidote üblichen Methoden ausgebracht werden. So können die erfindungsgemäßen Gegenmittel

entweder vor oder nach dem Herbizid ausgebracht oder zusammen mit dem Herbizid appliziert werden. Ferner können Kulturpflanzen durch Saatgutbehandlung mit den Gegenmitteln vor der Saat (Beizung) vor Schäden geschützt werden, wenn das Herbizid vor oder nach der Saat angewendet wird. Eine weitere Einsatzmöglichkeit besteht darin, daß man die Gegenmittel bei der Aussaat in die Saatfurche ausbringt. Wenn es sich bei den Pflanzen um Stecklinge handelt, so können diese vor der Auspflanzung mit den Gegenmitteln behandelt werden.

Beim Einsatz der erfindungsgemäßen Gegenmittel kommen die ortsüblichen Aufwandmengen an den jeweiligen Herbiziden zur Anwendung. Die Aufwandmengen an herbizidem Wirkstoff schwanken zwischen 0,5 und 5 kg/ha. Die Aufwandmenge an Gegenmittel ist unabhängig vom Herbizid und der Aufwandmenge des herbiziden Wirkstoffes. Im allgemeinen liegen die Aufwandmengen an erfindungsgemäßen Gegenmitteln bei Flächenbehandlung zwischen 0,1 und 5 kg/ha, vorzugsweise zwischen 0,2 und 4 kg/ha. Bei der Saatgutbehandlung liegen die Aufwandmengen an erfindungsgemäßen Gegenmitteln im allgemeinen zwischen 10 und 300 g pro Kilogramm Saatgut, vorzugsweise zwischen 25 und 200 g pro Kilogramm Saatgut.

In den erfindungsgemäßen Wirkstoffkombinationen können die Gewichtsverhältnisse von Gegenmitteln zu herbiziden Wirkstoffen in relativ großen Bereichen schwanken. Im allgemeinen entfallen auf 1 Gew.-Teil an herbizidem Wirkstoff 0,04 bis 1,0 Gew.-

Teile vorzugsweise 0,1 bis 0,5 Gew.-Teile an Gegenmittel der Formel (I).

Die Herstellung und die Verwendung der erfindungsgemäßen heterocyclisch substituierten Amide der Formel (I) geht aus den nachfolgenden Beispielen hervor.

## Herstellungsbeispiele

### Beispiel 1

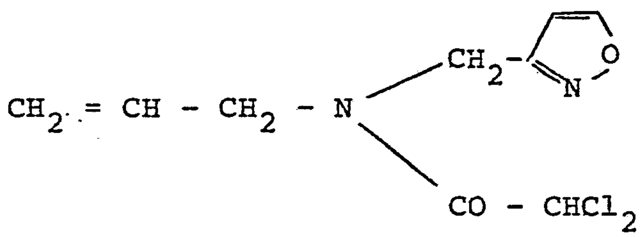

7 g (0,05 Mol) N-(Isoxazol-3-yl-methyl)-allylamin und 4,4 g (0,055 Mol) Pyridin werden in wasserfreiem Toluol gelöst und bei 20 bis 30°C tropfenweise mit 8,1 g (0,055 Mol) Dichloracetylchlorid versetzt. Man rührt noch eine Stunde nach und arbeitet dann auf, indem man die organische Phase abtrennt, mit Wasser wäscht, über Natriumsulfat trocknet und dann einengt. Der verbleibende Rückstand wird unter vermindertem Druck destilliert. Man erhält auf diese Weise 10,3 g (82 % der Theorie) an N-(Isoxazol-3-yl-methyl)-N-allyl-dichloracetamid.

Kp: 120°C / 0,1 Torr

$n_D^{20}$ = 1,5256

Herstellung des Vorproduktes:

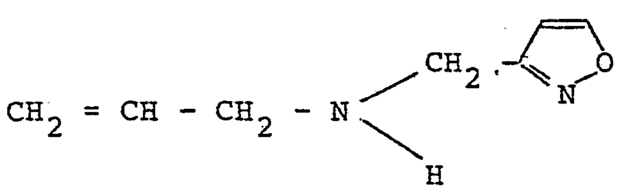

(II - 1)

171,3 g (3 Mol) Allylamin werden mit 200 ml Wasser gemischt und bei 5°C tropfenweise mit 117,5 g (1 Mol) 3-Chlormethyl-isoxazol versetzt. Man rührt noch 10 Stunden

bei 20°C nach und arbeitet dann auf, indem man das Reaktionsgemisch mit wässriger Natronlauge schwach alkalisch einstellt, mit Methylenchlorid extrahiert, die vereinigten organischen Phasen über Natriumsulfat trocknet und unter vermindertem Druck einengt. Der verbleibende Rückstand wird unter stark vermindertem Druck destilliert. Man erhält auf diese Weise 100,5 g (73 % der Theorie) an N-(Isoxazol-3-yl-methyl)-allylamin.
Kp = 98-100°C / 13 Torr

Beispiel 2

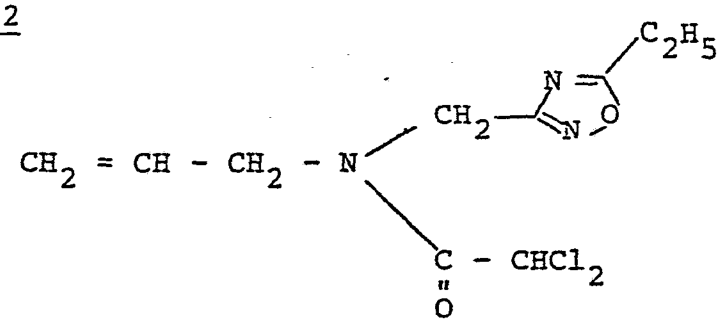

8,35 g (0,05 Mol) N-(5-Ethyl-(1,2,4-oxadiazol-3-yl)-methyl) allylamin und 3,95 g (0,05 Mol) Pyridin werden in 50 ml absolutem Tetrahydrofuran gelöst und bei Raumtemperatur tropfenweise mit 7,3 g (0,05 Mol) Dichloracetylchlorid versetzt. Man rührt noch eine Stunde bei Raumtemperatur nach und arbeitet dann auf, indem man das Reaktionsgemisch filtriert, einengt und in Methylenchlorid aufnimmt. Die organische Phase wird mit Wasser gewaschen, über Natriumsulfat getrocknet und eingeengt. Der verbleibende Rückstand wird in einer Kugelrohr-Destillationsapparatur gereinigt. Man erhält auf diese Weise 11,5 g (82,7 % der Theorie) an N-(5-Ethyl-(1,2,4-oxadiazol-3-yl)-methyl)-N-allyl-dichloracetamid.

Herstellung des Vorproduktes:

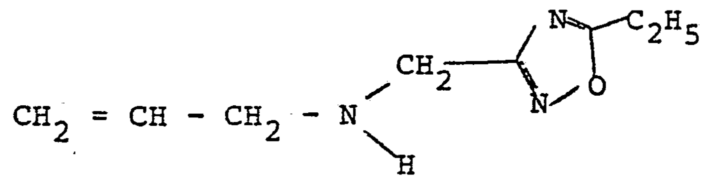 (II-2)

58,14 g (1,02 Mol) Allylamin werden mit 34 ml Wasser vermischt und bei 5 - 10°C unter Rühren tropfenweise mit 49,5 g (0,34 Mol) 3-Chlormethyl-5-ethyl-1,2,4-oxadiazol versetzt. Man läßt über Nacht bei Raumtemperatur nachrühren und arbeitet dann auf, indem man das Reaktionsgemisch mit wässriger Natronlauge alkalisch einstellt, mit Methylenchlorid extrahiert, die vereinigten organischen Phasen dreimal mit Wasser wäscht, über Natriumsulfat trocknet und dann einengt. Der verbleibende Rückstand wird unter vermindertem Druck destilliert. Man erhält auf diese Weise 37,2 g (65,5 % der Theorie) an N-(5-Ethyl-(1,2,4-oxadiazol-3-yl)-methyl)-allylamin.
Kp. = 106-111°C / 12 Torr

Beispiel 3

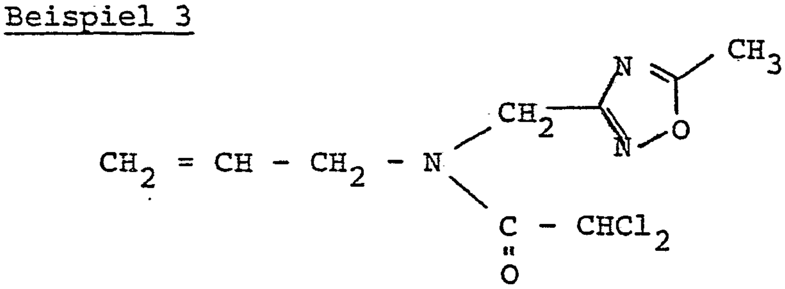

4,0 g (0,026 Mol) N-(5-Methyl-(1,2,4-oxadiazol-3-yl)-methyl)-allylamin und 2,05 g (0,026 Mol) Pyridin werden in 50 ml absolutem Tetrahydrofuran gelöst und bei Raumtemperatur tropfenweise mit 3,8 g (0,026 Mol) Dichloracetylchlorid versetzt. Man rührt noch eine Stunde bei Raumtemperatur nach und arbeitet dann auf, indem man das Reaktionsgemisch filtriert, einengt und in Methylenchlorid aufnimmt. Die organische Phase wird mit Wasser gewaschen, über Natriumsulfat getrocknet und eingeengt. Der verbleibende Rückstand wird in einer Kugelrohr-Destillationsapparatur gereinigt. Man erhält auf diese Weise 4,6 g (67 % der Theorie) an N-(5-Methyl-(1,2,4-oxadiazol-3-yl)-methyl)-N-allyl-dichloracetamid.

Kp = 220°C / 0,4 Torr

Herstellung des Vorproduktes:

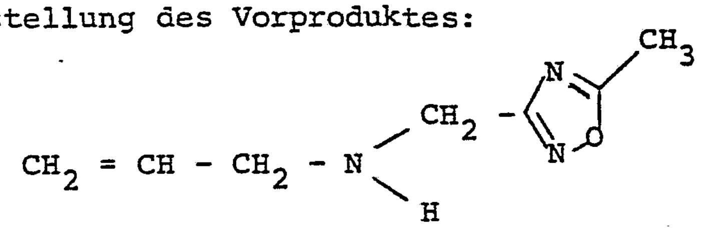

(II-3)

25,65 g (0,45 Mol) Allylamin werden mit 15 ml Wasser vermischt und bei 5 - 10°C unter Rühren tropfenweise mit 19,7 g (0,15 Mol) 3-Chlormethyl-5-methyl-1,2,4-oxadiazol versetzt. Man läßt über Nacht bei Raumtemperatur nachrühren und arbeitet dann auf, indem man das Reaktionsgemisch mit wässriger Natronlauge alkalisch einstellt, mit Methylenchlorid extrahiert, die vereinigten organischen Phasen dreimal mit Wasser wäscht, über Natriumsulfat trocknet und dann einengt. Der verbleibende Rückstand wird in einer Kugelrohr-Destillationsapparatur gereinigt. Man erhält auf diese Weise 12,4 g (54 % der Theorie) an N-(5-Methyl-(1,2,4-oxadiazol-3-yl)-methyl)-allylamin.

Kp = 200°C / 12 Torr

Beispiel 4

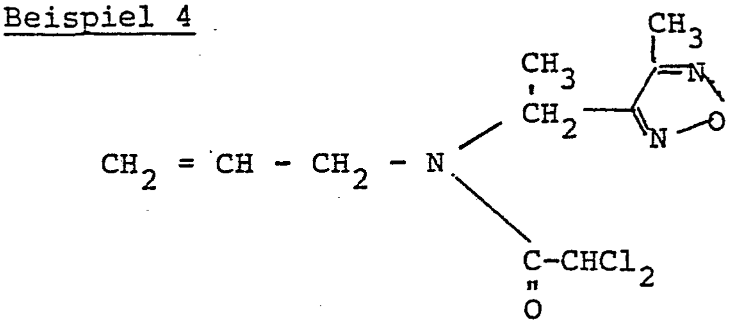

9 g (0,054 Mol) N-(4-Methyl-(1,2,5-oxadiazol-3-yl)-eth-1-yl)-allylamin und 4,3 g (0,054 Mol) Pyridin werden in 60 ml absolutem Tetrahydrofuran gelöst und bei Raumtemperatur tropfenweise mit 7,96 g (0,054 Mol) Dichloracetylchlorid versetzt. Man rührt noch eine Stunde bei Raumtemperatur nach und arbeitet dann auf, indem man das Reaktionsgemisch filtriert, einengt und in Methylenchlorid aufnimmt. Die organische Phase wird mit Wasser gewaschen, über Natriumsulfat getrocknet und eingeengt. Der verbleibende Rückstand wird in einer Kugelrohr-Destillationsapparatur gereinigt. Man erhält auf diese Weise 14,0 g (93 % der Theorie) an N-(4-Methyl-(1,2,5-oxadiazol-3-yl)-eth-1-yl)-N-allyl-dichloracetamid.
Kp = 190°C / 2 Torr

Herstellung des Vorproduktes:

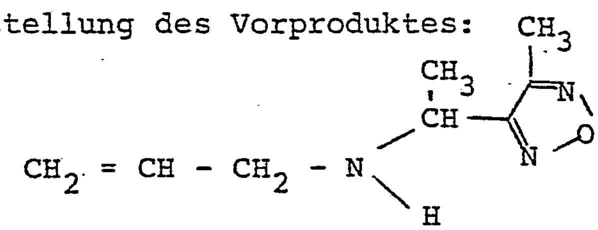

(II-4)

34,3 g (0,6 Mol) Allylamin werden mit 20 ml Wasser vermischt und bei 5-10°C unter Rühren tropfenweise mit 49,5g

(0,34 Mol) 3-Brom-eth-1-yl-4-methyl-1,2,5-oxadiazol versetzt. Man läßt über Nacht bei Raumtemperatur nachrühren und arbeitet dann auf, indem man das Reaktionsgemisch mit wässriger Natronlauge alkalisch einstellt, mit Methylenchlorid extrahiert, die vereinigten organischen Phasen dreimal mit Wasser wäscht, über Natriumsulfat trocknet und dann einengt. Der verbleibende Rückstand wird unter vermindertem Druck destilliert. Man erhält auf diese Weise 27,1 g (81,1 % der Theorie) an N-(4-Methyl-(1,2,5-oxadiazol-3-yl)-eth-1-yl)-allylamin. Kp = 200°C / 10 Torr

Beispiel 5

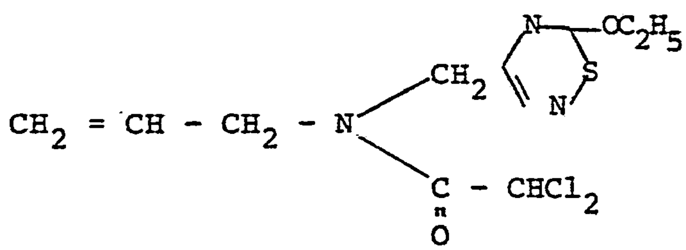

4,55 g (0,023 Mol) N-(5-Ethoxy-(1,2,4-thiadiazol-3-yl)-methyl)-allylamin und 1,82 g (0,023 Mol) Pyridin werden in 50 ml absolutem Tetrahydrofuran gelöst und bei Raumtemperatur tropfenweise mit 3,35 g (0,023 Mol) Dichloracetylchlorid versetzt. Man rührt noch eine Stunde bei Raumtemperatur nach und arbeitet dann auf, indem man das Reaktionsgemisch filtriert, einengt und in Methylenchlorid aufnimmt. Die organische Phase wird mit Wasser gewaschen, über Natriumsulfat getrocknet und eingeengt. Der verbleibende Rückstand wird in einer Kugelrohr-Destillationsapparatur gereinigt. Man erhält auf diese Weise 5,3 g (74,3 % der Theorie) an N-/‾5-Ethoxy-(1,2,4-thiadiazol-3-yl]-methyl)-N-allyl-dichloracetamid.

$n_D^{20}$ = 1,5589

Herstellung des Vorproduktes:

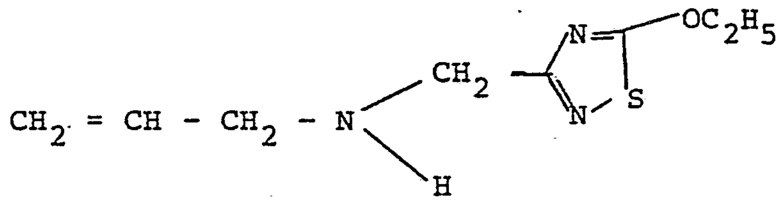

(II-5)

14,66 g (0,26 Mol) Allylamin werden mit 8,6 ml Wasser vermischt und bei 5 - 10°C unter Rühren tropfenweise mit 15,3 g (0,086 Mol) 3-Chlormethyl-(5-ethoxy-1,2,4-thiadiazol versetzt. Man läßt über Nacht bei Raumtemperatur nachrühren und arbeitet dann auf, indem man das Reaktionsgemisch mit wäßriger Natronlauge alkalisch einstellt, mit Methylenchlorid extrahiert, die vereinigten organischen Phasen dreimal mit Wasser wäscht, über Natriumsulfat trocknet und dann einengt. Der verbleibende Rückstand wird in einer Kugelrohr-Destillationsapparatur gereinigt. Man erhält auf diese Weise 9,1 g (53,2 % der Theorie) an N-(5-Ethoxy-(1,2,4-thiadiazol-3-yl)-methyl)-allylamin.

Kp = 110°C / 0,08 Torr

Beispiel 6

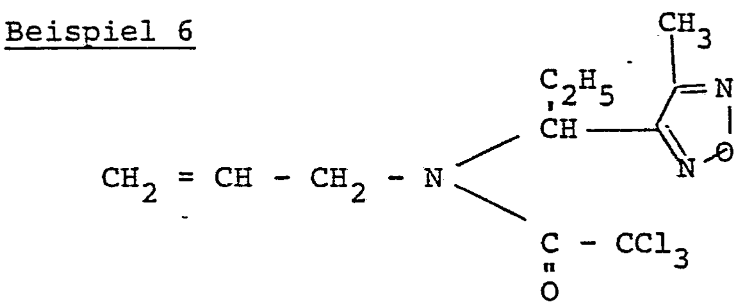

5 g (0,028 Mol) N-(4-Methyl-(1,2,5-oxadiazol-3-yl)-ethyl-methyl)-allylamin und 2 g (0,028 Mol) Pyridin werden in

50 ml absolutem Tetrahydrofuran gelöst und bei Raumtemperatur tropfenweise mit 5,1 g (0,028 Mol) Trichloracetylchlorid versetzt. Man rührt noch eine Stunde bei Raumtemperatur nach und arbeitet dann auf, indem man das Reaktionsgemisch filtriert, einengt und in Methylenchlorid aufnimmt. Die organische Phase wird mit Wasser gewaschen, über Natriumsulfat getrocknet und eingeengt. Der verbleibende Rückstand wird in einer Kugelrohr-Destillationsapparatur gereinigt. Man erhält auf diese Weise 5,4 g (85 % der Theorie) an N-(4-Methyl-(1,2,5-oxadiazol-3-yl)-ethylmethyl)-N-allyl-trichloracetamid.
Siedebeginn: 120°C / 0.07 Torr

Herstellung des Vorproduktes:

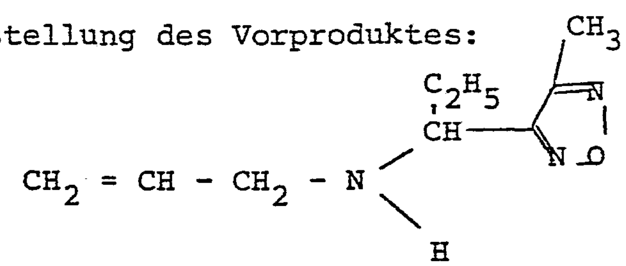

(II-6)

7,2 g (0,126 Mol) Allylamin werden mit 4,2 ml Wasser vermischt und bei 5 - 10°C unter Rühren tropfenweise mit 8,5 g (0,041 Mol) 3-Brom-ethylmethyl-4-methyl-1,2,5-oxadiazol versetzt. Man läßt über Nacht bei Raumtemperatur nachrühren und arbeitet dann auf, indem man das Reaktionsgemisch mit wässriger Natronlauge alkalisch einstellt, mit Methylenchlorid extrahiert, die vereinigten organischen Phasen dreimal mit Wasser wäscht, über Natriumsulfat trocknet und dann einengt. Der verbleibende Rückstand wird unter vermindertem Druck destilliert. Man erhält auf diese Weise 5,2 g (70, 1 % der Theorie) an N-(4-Methyl-(1,2,5-oxadiazol-3-yl)-ethylmethyl)-allylamin.

Beispiel 7

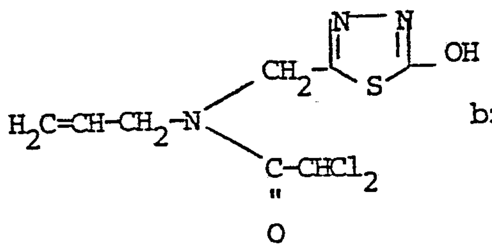 bzw. 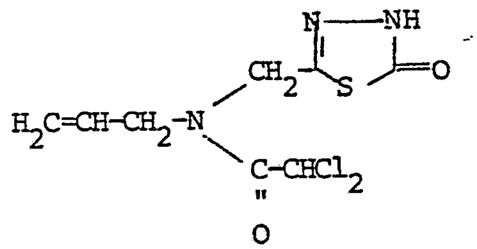

In eine Lösung von 9,97 g (0,05 Mol) N-(5-Ethoxy-1,2,4-thiadiazol-2-ylmethyl)-allylamin und 1 Tropfen Dimethylformamid in 50 ml Essigester werden bei 80°C in 25 Minuten 8,28 g (0,055 Mol) 98 prozentiges Dichloracetylchlorid eingetropft. Es wird 5,5 Stunden bei 80°C nachgerührt. Nach Abkühlung verdünnt man mit 50 ml Essigester, wäscht mit verdünnter Salzsäure, gesättigter Natriumhydrogencarbonat-Lösung und Wasser, trocknet über Natriumsulfat und dampft ein. Den teilweise kristallinen Rückstand preßt man auf einer Tonplatte ab und kristallisiert aus Essigester/Petrolether (1:1) um. Man erhält so 3,7 g (26,2 % der Theorie) an N-Allyl-N-(2(3H)-1,3,4-thiadiazolon-5-yl-methyl)-dichloracetamid vom Schmelzpunkt 93°C.

Herstellung des Vorproduktes:

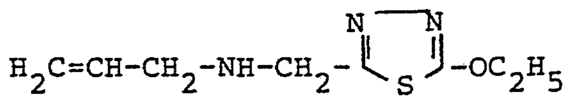 (II-7)

Eine Mischung von 78,7 g (1,38 Mol) Allylamin, 82,2 g (0,46 Mol) 2-Chlormethyl-5-ethoxy-1,3,4-thiadiazol, 63,5 g (0,46 Mol) Kaliumcarbonat und 700 ml Acetonitril wird 5 Stunden unter Rückfluß erhitzt. Nach Zusatz von etwas Aktivkohle wird noch einmal kurz aufgekocht, filtriert und das Filtrat im Vakuum eingedampft. Man erhält so 85,4 g (93,2% der Theorie) N-(5-Ethoxy-1,3,4-thiadiazol-2-ylmethyl)-allylamin als orangefarbenes Öl mit dem Brechungsindex $n_D^{20}$ : 1,5137.

Nach den in den Beispielen 1 bis 7 beschriebenen Methoden werden auch die in der folgenden Tabelle 3 formelmäßig aufgeführten erfindungsgemäßen Stoffe hergestellt:

Tabelle 3

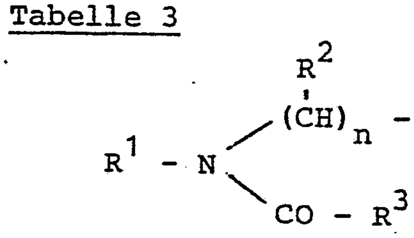 (I)

| Beispiel Nr. | $R^1$ | $R^2$ | n | $R^3$ | Het | Siedepunkt (°C/Torr) bzw. Brechungsindex ($n_D^{20}$) |
|---|---|---|---|---|---|---|
| 8 | $CH_2=CH-CH_2-$ | H | 1 | $-CCl_3$ | 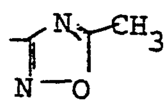 | 1,5187 |
| 9 | $CH_2=CH-CH_2-$ | H | 1 | $-CCl_3$ | 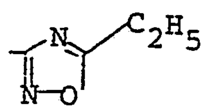 | Öl |
| 10 | $CH_2=CH-CH_2$ | H | 1 | $-CHCl_2$ | 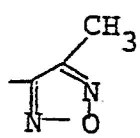 | 140/0,1 |
| 11 | $CH_2=CH-CH_2$ | H | 1 | $-CCl_3$ | 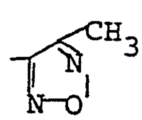 | 190/0,2 |

Tabelle 3 (Fortsetzung)

| Beispiel | $R^1$ | $R^2$ | n | $R^3$ | Het | Siedepunkt (°C/Torr) bzw. Brechungsindex ($n_D^{20}$) |
|---|---|---|---|---|---|---|
| 12 | $CH_2=CH-CH_2$ | $C_2H_5$ | 1 | $-CHCl_2$ | 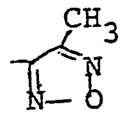 | 200/1 |
| 13 | $CH_2=CH-CH_2-$ | $CH_3$ | 1 | $-CCl_3$ | 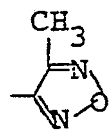 | 175/0,5 |
| 14 | $CH_2=CH-CH_2$ | H | 1 | $-CHCl_2$ | 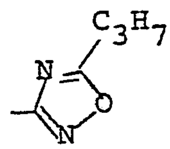 | Öl |
| 15 | $CH_2=CH-CH_2-$ | H | 1 | $-CCl_3$ | 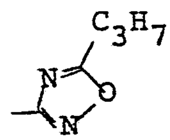 | Öl |
| 16 | $CH_2=CH-CH_2-$ | H | 1 | $-CCl_3$ | 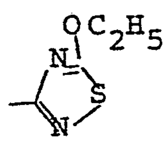 | Öl |
| 17 | $CH_2=CH-CH_2-$ | H | 1 | $-CHCl_2$ | 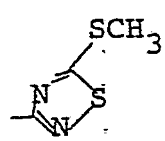 | Öl |
| 18 | $CH_2=CH-CH_2-$ | H | 1 | $-CCl_3$ | 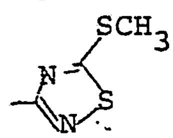 | Öl |

Tabelle 3 (Fortsetzung)

| Beispiel | $R^1$ | $R^2$ | n | $R^3$ | Het | Siedepunkt (°C/Torr) bzw. Brechungsindex ($n_D^{20}$) |
|---|---|---|---|---|---|---|
| 19 | $CH_2=CH-CH_2-$ | H | 1 | $-CHCl_2$ | 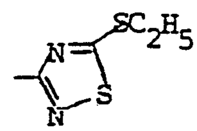 | Öl |
| 20 | $CH_2=CH-CH_2-$ | H | 1 | $-\underset{Cl}{CH}-CH_3$ | 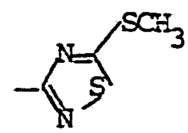 | Öl |
| 21 | $CH_2=CH-CH_2-$ | H | 1 | $-CHCl_2$ | 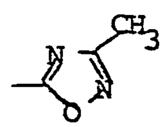 | 150/0,2 |
| 22 | $CH_2=CH-CH_2-$ | H | 1 | $-CHCl_2$ | 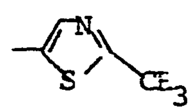 | Öl |
| 23 | $CH_2=CH-CH_2-$ | H | 1 | $-CH_2-O-CO-CH_3$ | 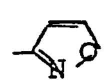 | 130/0,1 |
| 24 | $CH_2=CH-CH_2-$ | H | 1 | $-\underset{Cl}{CH}-CH_3$ | 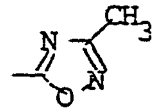 | 120/0,1 |
| 25 | $CH_2=CH-CH_2-$ | H | 1 | $-CHCl_2$ | 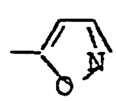 | 182-185/ 0,2 |

Tabelle 3 (Fortsetzung)

| Bei-spiel | $R^1$ | $R^2$ | n | $R^3$ | Het | Siedepunkt (°C/Torr) bzw. Brechungsindex ($n_D^{20}$) |
|---|---|---|---|---|---|---|
| 26 | $CH_2=CH-CH_2-$ | H | 1 | $-CH_2-OH$ | 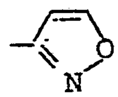 | 1,5253 |
| 27 | $CH_2=CH-CH_2-$ | H | 1 | $-CHCl_2$ | 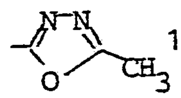 | 1,5205 |
| 28 | $CH_3-CH_2-CH_2-$ | H | 1 | $-CHCl_2$ | 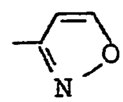 | 1,5135 |
| 29 | $CH_2=CH-CH_2-$ | H | 1 | $-CH_2-O-CH_3$ | 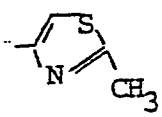 | 1,5309 |
| 30 | $CH_2=CH-CH_2-$ | H | 1 | $-CHCl_2$ | 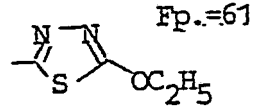 | Fp.=61-64°C |
| 31 | $CH_2=CH-CH_2-$ | - | 0 | $-CH(Cl)-CH_3$ | 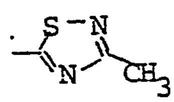 | Fp.=42°C |
| 32 | $CH_2=CH-CH_2$ | - | 0 | $-CHCl_2$ | 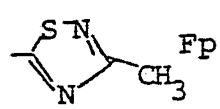 | Fp.=173°C |
| 33 | $CH_2=CH-CH_2-$ | H | 1 | $-CH(Cl)-CH_3$ | 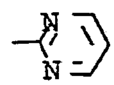 | Fp.=84°C |

Tabelle 3 (Fortsetzung)

| Beispiel | $R^1$ | $R^2$ | n | $R^3$ | Het | Siedepunkt (°C/Torr) bzw. Brechungsindex ($n_D^{20}$) |
|---|---|---|---|---|---|---|
| 34 | $CH_2=CH-CH_2-$ | H | 1 | $-\underset{Cl}{CH}-CH_3$ | 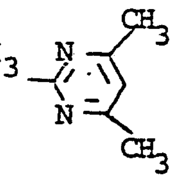 | öl |
| 35 | $CH_2=CH-CH_2-$ | H | 1 | $-CHCl_2$ | 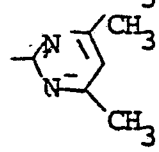 | öl |
| 36 | $CH_2=CH-CH_2-$ | H | 1 | $-CHCl_2$ | 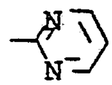 | Fp.=83°C |
| 37 | $CH_2=CH-CH_2-$ | H | 2 | $-CHCl_2$ | 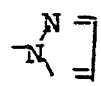 | öl |
| 38 | $CH_2=CH-CH_2-$ | H | 1 | $-CH_2CH_2Cl$ | 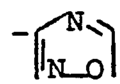 $-C_2H_5$ | öl |
| 39 | 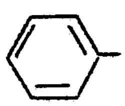 | H | 1 | $-CHCl_2$ | 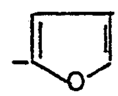 | Fp-64-65 °C |

Nach den in den Beispielen 1 bis 7 beschriebenen Methoden werden auch die in der folgenden Tabelle 4 formelmäßig aufgeführten Amine der Formel (II) hergestellt:

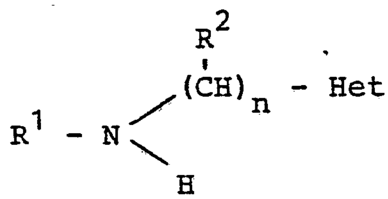

(II)

Tabelle 4

| Beispiel Nr. | $R^1$ | $R^2$ | n | Het |
|---|---|---|---|---|
| II- 8 | $CH_2$=CH-$CH_2$- | H | 1 | 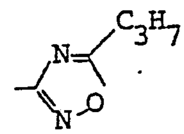 |
| II- 9 | $CH_2$=CH-$CH_2$- | $C_2H_5$ | 1 | 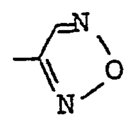 |
| II-10 | $CH_2$=CH-$CH_2$- | H | 1 | 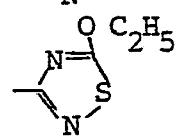 |
| II-11 | $CH_2$=CH-$CH_2$- | H | 1 | 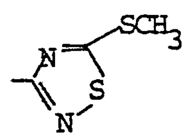 |
| II-12 | $CH_2$=CH-$CH_2$- | H | 1 | 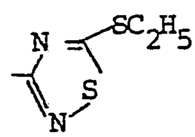 |
| II-13 | $CH_2$=CH-$CH_2$- | H | 1 | 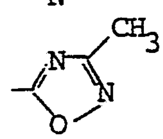 |
| II-14 | $CH_2$=CH-$CH_2$- | H | 1 | 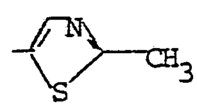 |
| II-15 | $CH_2$=CH-$CH_2$- | H | 1 | 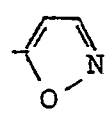 |

Tabelle 4

| Bei-spiel Nr. | $R^1$ | $R^2$ | n | Het |
|---|---|---|---|---|
| II-16 | $CH_2=CH-CH_2$ | H | 1 | 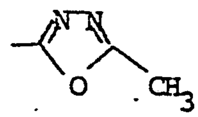 |
| II-17 | $CH_3-CH_2-CH_2-$ | H | 1 | 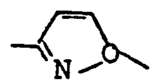 |
| II-18 | $CH_2=CH-CH_2-$ | H | 1 | 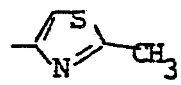 |
| II-19 | $CH_2=CH-CH_2-$ | - | 0 | 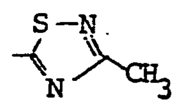 |
| II-20 | $CH_2=CH-CH_2-$ | H | 1 | 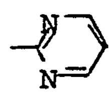 |
| II-21 | $CH_2=CH-CH_2-$ | H | 1 | 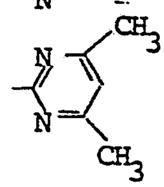 |
| II-22 | $CH_2=CH-CH_2-$ | H | 2 | 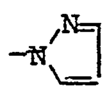 |
| II-23 | 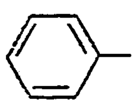 | H | 1 | 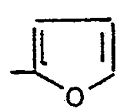 |

Die gute Wirksamkeit der erfindungsgemäßen Gegenmittel geht aus den nachfolgenden Beispielen hervor.

In diesem Beispiel werden die nachstehend angegebenen Verbindungen als Vergleichskomponenten eingesetzt:

(A) =

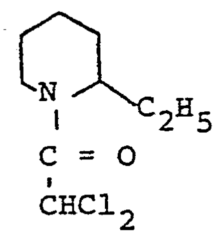

(N-Dichloracetyl-2-ethyl-piperidin)

Ferner wird in diesem Beispiel als herbizider Wirkstoff das nachstehend angegebene Acetanilid eingesetzt:

(B) =

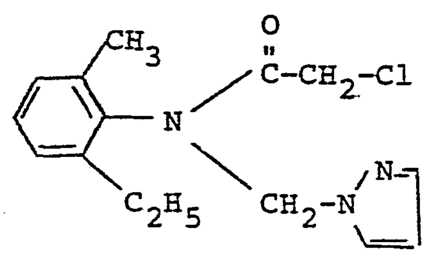

(2-Methyl-6-ethyl-N-(pyrazol-1-yl-methyl)-chloracetanilid).

Beispiel A

Pre-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gew.-Teil herbiziden Wirkstoff bzw. Gegenmittel bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 Stunden mit der Herbizid-Zubereitung bzw. mit der Gegenmittel-Zubereitung bzw. mit der Zubereitung aus Gegenmittel und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)

100 % = totale Vernichtung

Eine Auswertung der Testergebnisse zeigt, daß die erfindungsgemäßen Verbindungen (3) und (36) besser zum Schutz von Kulturpflanzen vor Schäden durch das 2-Methyl-6-ethyl-N-(pyrazol-1-yl-methyl)-chlor-acetanilid geeignet ist als die Vergleichskomponente (A).

Patentansprüche

1. Heterocyclisch substituierte Amide der Formel

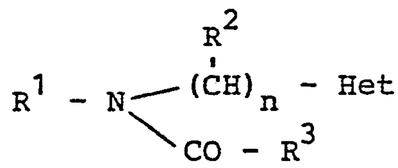

(I)

in welcher

$R^1$ für Alkyl, Alkenyl, Alkinyl, Halogenalkyl, Halogenalkenyl oder Alkoxyalkyl steht,

$R^2$ für Wasserstoff oder Alkyl steht,

$R^3$ für Dihalogenmethyl, Trihalogenmethyl, Halogenalkyl mit mehr als einem Kohlenstoffatom oder für den Rest $-CH_2-O-R^4$ steht,

worin

$R^4$ für Alkylcarbonyl, gegebenenfalls substituiertes Phenylcarbonyl, Alkylsulfonyl, gegebenenfalls substituiertes Phenylsulfonyl, Alkyl, Alkenyl, Alkinyl, Alkoxyalkyl oder Wasserstoff steht,

Het für einen gegebenenfalls substituierten ungesättigten oder mesoionischen Heterocyclus mit 1 bis 3 Heteroatomen steht, wobei als Heteroatome Sauerstoff, Schwefel und Stickstoff in Frage kommen und als Substituenten Alkyl, Alkoxy, Alkylthio, Alkylsulfinyl, Alkylsulfonyl, gegebenenfalls substituiertes Phenyl, Halogen, Hydroxy, Oxo, Mercapto, Thiono, Amino, Imino, Alkylamino, Alkylimino und/oder Dialkylamino in Betracht kommen, und

n für 0, 1 oder 2 steht,

oder

$R^1$ auch für Phenyl steht, wenn $R^2$ für Wasserstoff steht, $R^3$ für Dichlormethyl steht, Het für Fur-2-yl steht und n für 1 steht.

2) Heterocyclisch substituierte Amide der Formel (I) in denen $R^1$ für Alkyl mit 1 bis 6 Kohlenstoffatomen, Alkenyl mit bis zu 6 Kohlenstoffatomen, Alkinyl mit bis zu 6 Kohlenstoffatomen, Halogenalkyl mit bis zu 6 Kohlenstoffatomen und bis zu 5 Halogenatomen, Halogenalkenyl mit bis zu 6 Kohlenstoffatomen und bis zu 5 Halogenatomen, Alkoxyalkyl mit 1 bis 6 Kohlenstoffatomen in der Alkylgruppe und 1 bis 6 Kohlenstoffatomen in der Alkoxygruppe steht; oder $R^1$ auch für Phenyl steht, wenn $R^2$ für Wasserstoff steht, $R^3$ für Dichlormethyl steht, Het für Fur-2-yl steht und n für 1 steht; $R^2$ für Wasserstoff oder Alkyl mit 1 bis 4 Kohlenstoffatomen steht; n für 0, 1 oder 2 steht; $R^3$ für Dichlormethyl, Dibrommethyl, Trichlormethyl, Tribrommethyl, Halogenalkyl mit 2 bis 6 Kohlenstoffatomen und bis zu 5 Halogenatomen steht oder für den Rest $-CH_2-O-R^4$ steht, wobei $R^4$ für Alkylcarbonyl mit bis zu 6 Kohlenstoffatomen, gegebenenfalls durch Alkyl mit 1 bis 4 Kohlenstoffatomen und/oder Halogen substituiertes Phenylcarbonyl, Alkylsulfonyl mit 1 bis 6 Kohlenstoffatomen, gegebenenfalls durch Alkyl mit 1 bis 4 Kohlenstoffatomen und/oder Halogen substituiertes Phenylsulfonyl, Alkyl mit 1 bis 6 Kohlenstoffatomen, Alkenyl mit bis zu 6 Kohlenstoffatomen, Alkinyl mit bis zu 6 Kohlenstoffatomen, Alkoxyalkyl mit 1 bis 6 Kohlenstoffatomen in der Alkylgruppe und 1 bis 6 Kohlenstoffatomen in der Alkoxygruppe sowie für Wasserstoff steht; und Het für die über Stickstoff verknüpften Heterocyclen 1,2,4-Triazolyl, 1,2,3-Triazolyl, Pyrazolyl und Imidazolyl steht, die jeweils substituiert sein können durch Alkyl mit 1 bis 4 Kohlenstoffatomen, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, gegebenenfalls durch Halogen und/oder Alkyl mit 1 bis 4 Kohlenstoffatomen substituiertes Phenyl, Fluor, Chlor, Brom, Hydroxy, Oxo, Mercapto, Thiono, Amino, Imino, Alkylamino und Alkylimino mit jeweils 1 bis 4 Kohlenstoffatomen und/oder Dialkylamino mit 1 bis 4 Kohlenstoffatomen in jeder Alkylgruppe, und Het weiterhin für die über Kohlenstoff verknüpften Heterocyclen Pyrimidinyl, Oxazolyl, Isoxazolyl, Thiazolyl, Isothiazolyl, 1,2,4-Oxadiazolyl, 1,3,4-Oxadiazolyl, 1,2,5-Oxadiazolyl, 1,2,4-Thiadiazolyl, 1,3,4-Thiadiazolyl, 1,2,5-Thiadiazolyl, Imidazolyl, Pyrazolyl, 1,2,3-Triazolyl, 1,2,4-Triazolyl und Furyl steht, die jeweils substituiert sein können durch Alkyl mit 1 bis 4 Kohlenstoffatomen, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, gegebenenfalls durch Halogen und/oder Alkyl mit 1 bis 4 Kohlenstoffatomen substituiertes Phenyl, Fluor, Chlor, Brom, Hydroxy, Mercapto, Amino, Alkylamino mit 1 bis 4 Kohlenstoffatomen und/oder Dialkylamino mit 1 bis 4 Kohlenstoffatomen in jeder Alkylgruppe.

3. Verfahren zur Herstellung von heterocyclisch substituierten Amiden der Formel (I), dadurch gekennzeichnet, daß man

a) Amine der Formel

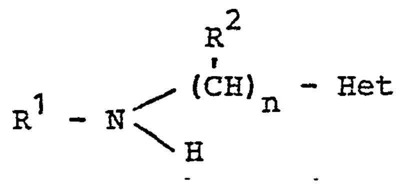

(II)

in welcher

$R^1$, $R^2$, Het und n die oben angegebene Bedeutung haben,

mit Acylhalogeniden der Formel

$$R^3 - CO - Y \qquad (III)$$

in welcher

Y für Chlor oder Brom steht und

$R^3$ die oben angegebene Bedeutung hat,

in Gegenwart eines Verdünnungsmittels und gegebenenfalls in Gegenwart eines Säurebindemittels umsetzt,

oder

b) Verbindungen der Formel

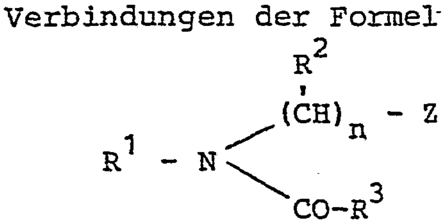

(IV)

in welcher

$R^1$, $R^2$, $R^3$ und n die oben angegebene Bedeutung haben und

Z für eine offenkettige Vorstufe des herzustellenden Heterocyclus steht,

gegebenenfalls in Gegenwart eines Verdünnungsmittels mit cyclisierenden Agenzien behandelt,

oder

c) Verbindungen der Formel

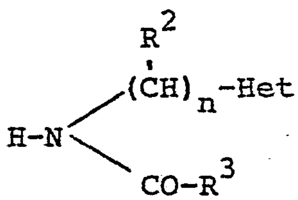

(V)

in welcher
$R^2$, $R^3$, Het und n die oben angegebene Bedeutung haben,
mit Halogenverbindungen der Formel

$R^1$-Y (VI)

in welcher
$R^1$ und Y die oben angegebene Bedeutung haben,
gegebenenfalls in Gegenwart eines Verdünnungsmittels sowie in Gegenwart eines Säurebindemittels umsetzt,

oder

d) Verbindungen der Formel

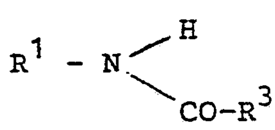

(VII )

in welcher
$R^1$ und $R^3$ die oben angegebene Bedeutung haben,

mit Verbindungen der Formel

$$Y-(\overset{R^2}{\overset{|}{C}}H)_n-Het \qquad (VIII)$$

in welcher
$R^2$, Y, n und Het die oben angegebene Bedeutung haben,

gegebenenfalls in Gegenwart eines Verdünnungsmittels sowie in Gegenwart eines Säurebindemittels umsetzt.

4) Mittel zum Schutz von Kulturpflanzen vor Schädigungen durch herbizid wirksame Thiolcarbamate bzw. durch herbizid wirksame Acetanilide, gekennzeichnet durch einen Gehalt an mindestens einem heterocyclisch substituierten Amid der Formel (I).

5) Verwendung von heterocyclisch substituierten Amiden der Formel (I) zum Schutz von Kulturpflanzen vor Schädigungen durch herbizid wirksame Thiolcarbamate bzw. durch herbizid wirksame Acetanilide.

6) Mittel zur selektiven Unkrautbekämpfung in Nutzpflanzenkulturen, gekennzeichnet durch einen Gehalt an einer Wirkstoffkombination, bestehend aus mindestens einem heterocyclisch substituierten Amid der Formel (I) und mindestens einem herbizid wirksamen Thiolcarbamat bzw. mindestens einem herbizid wirksamen Acetanilid.

7) Amine der Formel

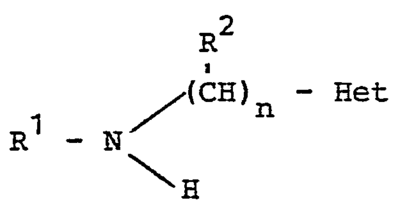

(II)

in welcher

$R^1$ für Alkyl, Alkenyl, Alkinyl, Halogenalkyl, Halogenalkenyl oder Alkoxyalkyl steht,

$R^2$ für Wasserstoff oder Alkyl steht;

Het für einen gegebenenfalls substituierten ungesättigten oder mesoionischen Heterocyclus mit 1 bis 3 Heteroatomen steht, wobei als Heteroatome Sauerstoff, Schwefel und Stickstoff in Frage kommen und als Substituenten Alkyl, Alkoxy, Alkylthio,

Alkylsulfinyl, Alkylsulfonyl, gegebenenfalls substituiertes Phenyl, Halogen, Hydroxy, Oxo, Mercapto, Thiono, Amino, Imino, Alkylamino und/oder Dialkylamino in Betracht kommen,
und

n für 0, 1 oder 2 steht,

oder

$R^1$ auch für Phenyl steht, wenn $R^2$ für Wasserstoff steht, Het für Fur-2-yl steht und n für 1 steht.

8) Verfahren zur Herstellung von Aminen der Formel (II) dadurch gekennzeichnet, daß man Amine der Formel

$$R^1 - NH_2 \qquad (IX)$$

in welcher
$R^1$ die oben angegebene Bedeutung hat,

mit Verbindungen der Formel

$$Y - (\overset{R^2}{\overset{|}{C}}H)_n - Het \qquad (VIII)$$

in welcher
$R^2$, Y, Het und n die oben angegebene Bedeutung haben,

gegebenenfalls in Gegenwart eines Säurebinders und gegebenenfalls in Gegenwart eines Verdünnungsmittels, oder in einem wäßrig-organischen Zweiphasensystem umsetzt.

9) Verbindungen der Formel

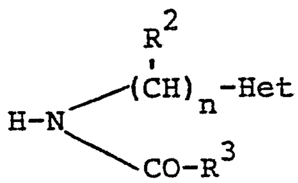

(V)

in welcher

$R^2$ für Wasserstoff oder Alkyl steht,

$R^3$ für Dihalogenmethyl, Trihalogenmethyl, Halogenalkyl mit mehr als einem Kohlenstoffatom oder für den Rest $-CH_2-O-R^4$ steht,

worin

$R^4$ für Alkylcarbonyl, gegebenenfalls substituiertes Phenylcarbonyl, Alkylsulfonyl, gegebenenfalls substituiertes Phenylsulfonyl, Alkyl, Alkenyl, Alkinyl, Alkoxyalkyl oder Wasserstoff steht,

Het für einen gegebenenfalls substituierten ungesättigten oder mesoionischen Heterocyclus mit 1 bis 3 Heteroatomen steht, wobei als Heteroatome Sauerstoff, Schwefel und Stickstoff in Frage kommen und als Substituenten Alkyl, Alkoxy, Alkylthio, Alkylsulfinyl, Alkylsulfonyl, gegebenenfalls substituiertes Phenyl, Halogen, Hydroxy, Oxo, Mercapto, Thiono, Amino, Imino, Alkylamino und/oder Dialkylamino in Betracht kommen,

und

n für 0, 1 oder 2 steht.

10) Verfahren zur Herstellung von Verbindungen der Formel (V), dadurch gekennzeichnet, daß man Amin-Derivate der Formel

$$H_2N-\overset{R^2}{\overset{|}{(CH)}}_n-Het \qquad (X)$$

in welcher
$R^2$, Het und n die oben angegebene Bedeutung haben,

mit Acylhalogeniden der Formel

$$R^3-CO-Y \qquad (III)$$

in welcher
$R^3$ und Y die oben angegebene Bedeutung haben,

in Gegenwart eines Verdünnungsmittels sowie gegebenenfalls in Gegenwart eines Säurebindemittels umsetzt.